# EUROPEAN PATENT SPECIFICATION

(11) **EP 2 181 104 B1**
(45) Date of publication and mention of the grant of the patent: **07.09.2011**
(21) Application number: 08796668.5
(22) Date of filing: 25.07.2008
(51) Int. Cl.: C07D 313/00, A61K 31/365, A61P 35/00

(54) **ZEARALENONE MACROLIDE DERIVATIVES AND USES OF THE SAME**
ZEARALENON-MAKROLIDDERIVATE UND IHRE VERWENDUNG
DÉRIVÉS DU MACROLIDE ZÉARALÉNONE ET LEURS UTILISATIONS

(30) Priority: 25.07.2007 US 951892 P
(43) Date of publication of application: 05.05.2010
(73) Proprietor: Eisai R&D Management Co., Ltd., Tokyo (JP)
(72) Inventor: DU, Hong, Andover, MA 01810 (US); WANG, John (Yuan), Andover, MA 01810 (US)
(74) Representative: Harris, Jennifer Lucy
(86) International application number: PCT/US2008/071248
(87) International publication number: WO 2009/015363

(56) References cited:
- WO-A-03/076424

## Description

### BACKGROUND OF THE INVENTION

F152 (LL-Z1640-2), a zearalenone-like macrolide, was first isolated from shake flask fermentation, crude extracts of which inhibited the ciliated protozoan *Tetrahymena pyriformis* (see, McGahren et al. J. Org. Chem. 1978, 43, 2339). Although initial biological 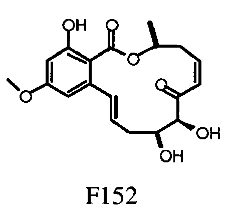 studies using this natural product failed to yield any particularly interesting activities, the possibility of preparing additional derivatives and/or further exploring their biological activity was undertaken. For example, F152 and certain isomers thereof inhibit the phosphorylating enzyme Mapk/Erk kinase (MEK) and may be useful for the treatment of certain MEK-related cancers and other diseases characterized by the formation of neoangiogenesis (see, *e.g*., GB 323 845). Derivatives of F152 have also been shown to have activity as tyrosine kinase inhibitors, which are useful, for example, for the treatment of cancer and inflammatory disorders (*see*, *e.g*., EP 606 044; WO 00/38674; JP 8-40893; WO 96/13259; 5,728,726; 5,674,892; 5,795,910). Often, however, F152 and derivatives thereof are obtained by fermentation techniques and modifications to the natural product and thus were limited in the number and types of derivatives that could be prepared and evaluated for biological activity. Additionally, although F152 and certain derivatives thereof have demonstrated potent *in vitro* activities, these compounds may be biologically unstable (for example, they are susceptible to enone isomerization in mouse and human plasma), thereby limiting the development of these compounds as therapeutics for the treatment of humans or other animals.

Recently, certain F152 analogues have been shown to be inhibitors of NF-κB activation, and MEK1 (See, *e.g*., U.S. Application Serial No.: 10/507,067 and U.S. Application Publication No.: US 2004/0224936). The compounds where also reported to inhibit AP-1 activation and some protein kinases (for example, MEKK, PDGFr, VEGFr). Based on these mechanisms of action, it was suggested that the compounds inhibit the production of various pro-inflammatory and/or immunologic cytokines such as TNFα, IL-1, IL-6, IL-8, IL-2 etc, and also inhibit the production of various pro-inflammatory molecules under the regulation of NF-κB pathway such as prostaglandins produced from COX-2, ICAM-1 and MMP-1 and 3 etc. Also, it was reported that the compounds have the ability to inhibit cell proliferation under the regulation of AP-1 pathway through the inhibition of MEK1. In addition, it was reported that the compounds have ability to inhibit angiogenesis, likely based on the inhibitory activity on VEGFr kinase and weak inhibitory activity on PDGFr kinase.

### SUMMARY OF THE INVENTION

The present invention is based, at least in part, on the discovery of novel zearalenone macrolide analogs which have a triple bond in the macrocyclic portion of the structure. Without wishing to be bound by any particular theory, it is believed that, because such compounds are active as inhibitors of MEK1 (as determined herein), they can be useful for the treatment of cancer.

Accordingly, in some aspects, the present invention provides compounds of formula (I) 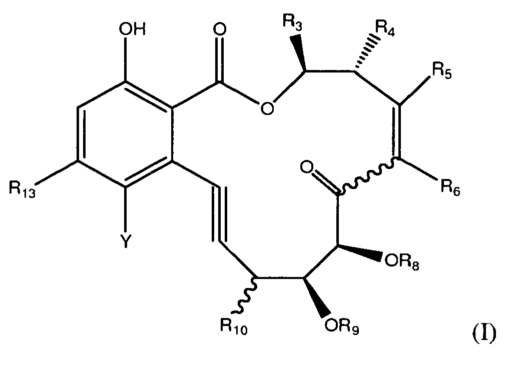 wherein
R₃ is a moiety selected from the group consisting of H and a C₁-₆ alkyl group;
R₄ is a C₁-₆ alkyl group, substituted with 0, 1, 2, or 3 halogen moieties;
R₅ is a hydrogen;
R₆ is a moiety selected from the group consisting of H and halogen; wherein R₅ and R₆ may be either cis or trans;
R₈ is H;
R₉ is a moiety selected from the group consisting of H and trifluoromethylcarbonyl; or R₈ and R₉ are taken together with the core structure to form a heterocyclydiyl of formula (a): 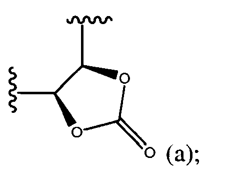
R₁₀ is a moiety selected from the group consisting of hydrogen and hydroxyl;
R₁₃ is a moiety selected from the group consisting of a C₁₋₆ alkylamino group and a C₁₋₆ alkoxy group, each of which is substituted with 0, 1, 2, or 3 hydroxyl moieties;
Y is a moiety selected from the group consisting of hydrogen, halogen and hydroxyl; and pharmaceutically acceptable salts and esters thereof.

In some embodiments, R₃ is methyl. In some embodiments, R₄ is a methyl or a trifluoromethyl. In some embodiments, R₆ is H or fluorine. In some embodiments, R₅ and R₆ are trans. In some embodiments, Y is H, F, Cl or Br. In some embodiments, R₁₃ is an unsubstituted C₁₋₆ alkylamino. In some embodiments, R₁₃ is selected from the group consisting of methylamino and ethylamino.

In some embodiments, the compounds of formula (I) include the following compounds: 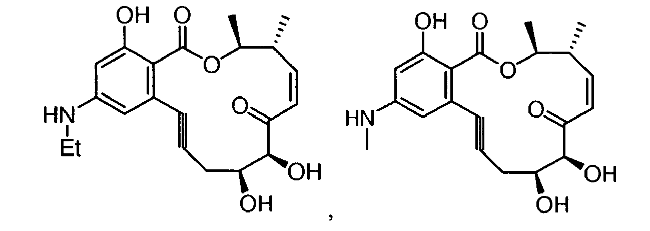 and pharmaceutically acceptable salts and esters thereof.

In some aspects, the present invention provides a composition comprising any of the compounds described herein, e.g., compounds of formula (I) and a pharmaceutically acceptable carrier. In some embodiments, the composition is suitable for oral administration. In some embodiments, the composition is suitable for intravenous administration.

In some aspects, the present invention provides methods for the treatment of cancer in a subject in need thereof comprising administering to the subject a composition in an amount effective to treat the cancer. In some embodiments, the cancer is a MEK1 associated cancer. In some embodiments, the cancer is a B-RAF mutated cancer. In some embodiments, the cancer is an NF-κB associated cancer. In some embodiments, the methods of the present invention further include administration of a second chemotherapeutic drug. In some embodiments, the methods of the present invention include administration of the composition at a dosage between about 0.10 mg/kg to about 25 mg/kg of body weight.

In some aspects, the present invention is directed to the use of a compound of formula (I) or a composition comprising a compound of formula (I) in therapy. In other aspects, the present invention is directed to the use of a compound of formula (I) or composition comprising a compound of formula (I) in the manufacture of a medicament for treating cancer.

### BRIEF DESCRIPTION OF THE DRAWINGS

**Figure 1** depicts a schematic representation of the RAS-MAPK signaling pathway.
**Figure 2** describes results of exemplary compounds of the invention in a biochemical MEK1 assay.

### DETAILED DESCRIPTION OF THE INVENTION

As discussed in WO 05/023792 and WO 03/076424, certain analogs of F152 have been reported to have inhibitory activity against MEK1, have demonstrated increased stability and are potent inhibitors of NF-κB activation, AP-1 activation and protein kinases (for example, MEKK, MEK1, PDGFr, VEGFr). The present invention is based, at least in part, on the discovery of a new class of F152 macrolide analogs, which include a triple bond in the macrocyclic portion of the compound, as described in more detail herein. Without wishing to be bound by any particular theory, it is believed that the compounds are stable inhibitors of the activities of MEK1 and/or NF-κB. Based on these mechanisms of action, the compounds of the present invention can be used for the treatment of cancers including, but not limited to solid tumor cancers, leukemias, lymphomas, and myelomas, such as chronic lymphocytic leukemia (CLL), acute myeloid leukemia (AML), B-cell lymphomas (e.g., non-Hodgkin's B-cell lymphomas), multiple myeloma, melanoma, ovarian cancer, thyroid cancer, pancreatic cancer, gliomas, such as astrocytomas, glioblastoma multiforme and other CNS tumors, colorectal cancer and/or breast cancer.

### Definitions

In order to more clearly and concisely describe the subject matter of the claims, the following definitions are intended to provide guidance as to the meaning of specific terms used herein.

It is to be noted that the singular forms "a," "an," and "the" as used herein include "at least one" and "one or more" unless stated otherwise. Thus, for example, reference to "a pharmacologically acceptable carriers" includes mixtures of two or more carriers as well as a single carrier, and the like.

When used herein, a specific protein kinase includes not only the naturally occurring kinase, *e.g*., MEK1 and other kinases mentioned specifically herein, but also modified kinases. Accordingly, in some embodiments, the compounds of the present invention inhibit kinases which share at least 90% sequence identity with naturally occurring counterparts. In other embodiments, the compounds of the present invention inhibit kinases which share at least 95% sequence identity with naturally occurring counterparts. In still other embodiments, the compounds of the present invention inhibit kinases which share at least 97% sequence identity with naturally occurring counterparts. In yet other embodiments, the compounds of the present invention inhibit kinases which share at least 99% sequence identity with naturally occurring counterparts. Kinases which share sequence identity with naturally occurring counterparts can be naturally occurring or synthetic. The term "sequence identity" generally refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base (e.g., A, T, C, G, U, or I, in the case of nucleic acids) occurs in both sequences to yield the number of matched positions, dividing the number of matched positions by the total number of positions in the region of comparison (*i.e*., the window size), and multiplying the result by 100 to yield the percentage of sequence identity. In some embodiments, the window size is at least 20 nucleotide positions, for example, at least 25-50 nucleotides. In other embodiments, the window size is greater than 50 nucleotides. In some embodiments, the window size is the entire naturally occurring protein kinase. In other embodiments, the window size is the sequence which corresponds at least to the ATP binding site on the naturally occurring protein kinase.

As used herein, the term "inhibit" when used in relation to a protein kinase, refers to the ability of a compound of the present invention to at least partially reduce the activity of the protein kinase. Without wishing to be bound by any particular theory, it is believed that an inhibitor of a protein kinase may either compete directly with ATP for the binding pocket of a specific kinase or otherwise interact with the kinase such that the binding pocket is no longer available to the ATP. The amount to which a compound will inhibit a protein kinase is ascertainable by a number of assays known in the art. For example, a compound may be diluted in an acceptable buffer (*e.g*., MOPS, EDTA, Brij-35, Glycerol, NaCl, BSA, HEPES, Triton X-100, or TRIS), and exposed to the protein kinase in the presence of a specific amount of ATP, *e.g*., in some cases [γ-³³P-ATP], and a substrate peptide. Following incubation and quenching, an aliquot will be taken to determine the amount of phosphate (*e.g*., γ-³³P) incorporated into the substrate peptide, which is associated with kinase activity. For example, ionizing radiation will be measured with a scintillation counter, to determine the amount of γ-³³P incorporated. This value would be compared to a suitable control, *e.g*., the same process without the inhibitor present. In some embodiments, the inhibitors of the present invention inhibit at least about 30% of the activity of the specified protein kinase at an inhibitor concentration of about 0.1 µM. In some embodiments, the inhibitors of the present invention inhibit at least about 45% of the activity of the specified protein kinase at an inhibitor concentration of about 0.1µM. In some embodiments, the inhibitors of the present invention inhibit at least about 50% of the activity of the specified protein kinase at an inhibitor concentration of about 0.1µM. In some embodiments, the inhibitors of the present invention inhibit at least about 55% of the activity of the specified protein kinase at an inhibitor concentration of about 0.1µM. In some embodiments, the inhibitors of the present invention inhibit at least about 40% of the activity of the specified protein kinase at an inhibitor concentration of about 1.0µM. In some embodiments, the inhibitors of the present invention inhibit at least about 50%, of the activity of the specified protein kinase at an inhibitor concentration of about 1.0µM. In some embodiments, the inhibitors of the present invention inhibit at least about 75%, 80%, 85%, or 90% of the activity of the specified protein kinase at an inhibitor concentration of about 1.0µM. In some embodiments, the inhibitors of the present invention inhibit about 95% of the activity of the specified protein kinase at an inhibitor concentration of about 1.0µM. In some embodiments, the assay to determine amount of inhibition is performed in the presence of ATP. In some embodiments, the assay to determine amount of inhibition is performed in the presence of 10 µM ATP. In some embodiments, the assay to determine amount of inhibition is performed in the presence of [γ-³³P-ATP]. In some embodiments, the assay to determine amount of inhibition is performed in the presence of an amount of [γ-³³P-ATP] which affords a specific activity of about 500 cpm/pmol. In some embodiments, the assay to determine amount of inhibition is performed in the presence of trace amounts of [γ-³³P-ATP].

"Treatment", or "treating" as used herein, is defined as the application or administration of a therapeutic agent (*e.g*., specific multikinase inhibitors of the invention) to a patient, or to an isolated tissue or cell line from a patient. The patient generally has a disease or disorder, a symptom of disease or disorder or a predisposition toward a disease or disorder. The purpose of treatment is generally to cure, heal, alleviate, relieve, remedy, ameliorate, or improve such disease, disorder, symptoms or predisposition. "Treated," as used herein, refers to the disease or disorder being cured, healed, alleviated, relieved, remedied, ameliorated, or improved. For example, methods of treatment of the instant invention provide for administration of an inhibitor as described herein, such that the progression of a specific cancer or a specific class of cancer (*e.g*., a MEK1 associated cancer) is slowed or stopped. Methods of treatment of the instant invention further include the administration of an inhibitor, such that a specific cancer is cured.

The term "effective dose" or "effective dosage" is defined as an amount sufficient to achieve a desired effect. The term "desired effect" refers generally to any result that is anticipated by the skilled artisan when a compound or composition of the present invention is administered to a subject. In some embodiments, the desired effect is a complete remission of the disease or disorder. In other embodiments, the desired effect is a partial treatment of a disease or disorder. In still other embodiments, the desired effect is a full or partial treatment of the symptoms of a disease or disorder. For example, in some embodiments, the desired effect is the shrinkage of a solid tumor. In another exemplary embodiment, the desired effect is the elimination of a solid tumor. The term "therapeutically effective dose" is defined as an amount sufficient to cure or at least partially arrest the disease and its complications in a subject already suffering from the disease.

The term "subject," as used herein, refers to animals such as mammals, including, but not limited to, humans, primates, cows, sheep, goats, horses, pigs, dogs, cats, rabbits, guinea pigs, rats, mice or other bovine, ovine, equine, canine, feline, rodent or murine species. In some embodiments, the subject is a human.

Numerous values and ranges are recited in connection with various embodiments of the present invention, *e.g*., amount of a compound of the invention present in a composition. It is to be understood that all values and ranges which fall between the values and ranges listed are intended to be encompassed by the present invention unless explicitly stated otherwise.

The term "stable", as used herein, generally refers to compounds which possess stability sufficient to allow manufacture and which maintain the integrity of the compound for a sufficient period of time to be detected. In some embodiments, stable compounds of the present invention are those which maintain their integrity for a sufficient period of time to be useful for the purposes detailed herein. For example, a prodrug may be considered "stable" even though it is eventually metabolized in the body.

As used herein, "alkyl" groups include saturated hydrocarbons having one or more carbon atoms, including straight-chain alkyl groups (*e.g*., methyl, ethyl, propyl, butyl, pentyl, hexyl, etc.), cyclic alkyl groups (or "cycloalkyl" or "alicyclic" or "carbocyclic" groups) (*e.g*., cyclopropyl, cyclopentyl, cyclohexyl, etc.), branched-chain alkyl groups (isopropyl, *ter*t-butyl, sec-butyl, isobutyl, etc.), and alkyl-substituted alkyl groups (*e.g*., alkyl-substituted cycloalkyl groups and cycloalkyl-substituted alkyl groups). In certain embodiments, a straight-chain or branched-chain alkyl group may have 8 or fewer carbon atoms in its backbone, *e.g*., C₁-C₈ for straight-chain or C₃-C₈ for branched-chain. In certain embodiments, a straight-chain or branched-chain alkyl group may have 6 or fewer carbon atoms in its backbone, *e.g*., C₁-C₆ for straight-chain or C₃-C₆ for branched-chain. In still other embodiments, an alkyl group includes about 1 to 4 carbons. In other embodiments, an alkyl group includes about 1 to 3 carbons. In yet other embodiments, an alkyl group includes about 1 or 2 carbons. The term "lower alkyl" refers to alkyl groups having from 1 to 6 carbons in the chain, and to cycloalkyl groups having from 3 to 6 carbons in the ring structure. The term "C₁-C₆" as in "C₁-C₆ alkyl" means alkyl groups containing 1 to 6 carbon atoms. The terms "alkenyl" and "alkynyl" refer to unsaturated aliphatic groups analogous to alkyls, but which contain at least one double or triple carbon-carbon bond respectively.

The term "amine" or "amino," as used herein, refers to an unsubstituted or substituted moiety of the formula -NR^{a}R^{b}, in which R^{a} and R^{b} are each independently hydrogen, alkyl, aryl, or heterocyclyl, or R^{a} and R^{b}, taken together with the nitrogen atom to which they are attached, form a cyclic moiety having from 3 to 8 atoms in the ring. Thus, the term amino includes cyclic amino moieties such as piperidinyl or pyrrolidinyl groups, unless otherwise stated.

Regarding connectivity, an "arylalkyl" group, for example, is an alkyl group substituted with an aryl group (*e.g*., phenylmethyl (*i.e*., benzyl)). An "alkylaryl" moiety is an aryl group substituted with an alkyl group (*e.g*., *p*-methylphenyl (*i.e., p*-tolyl)). Thus, the term imidazolyl-alkyl refers to an alkyl group substituted with an imidazolyl moiety.

When specified, the chemical moieties of the compounds of the invention, including those groups discussed above, may be "substituted or unsubstituted." In some embodiments, the term "substituted" means that the moiety has substituents placed on the moiety other than hydrogen (*i.e*., in most cases, replacing a hydrogen), which allow the molecule to perform its intended function. It will be understood that "substitution" or "substituted with" includes the implicit proviso that such substitution is in accordance with the permitted valence of the substituted atom and the substituent, and that the substitution results in a stable compound, *e.g*., which does not spontaneously undergo transformation such as by rearrangement, cyclization, elimination, etc. As used herein, the term "substituted" is meant to include all permissible substituents of organic compounds. In a broad aspect, permissible substituents include acyclic and cyclic, branched and unbranched, carbocyclic and heterocyclic, aromatic and nonaromatic substituents of organic compounds. The compounds of the present invention may have one or more substitutions, as described herein.

When compounded chemical names, *e.g*., "alkylaryl," "aryloxy," and the like, are used herein, they are understood to have a specific connectivity to the core of the chemical structure. The moiety listed farthest to the right (*e.g*., aryl in "alkylaryl"), is the moiety which is directly connected to the core. That is, for example, in a structure V-CH₂CH₂CH₃, when the variable "V" is an alkylaryl, the structure is understood to be alkyl-aryl-CH₂CH₂CH₃.

As used herein, the term "compound" is intended to mean a substance made up of molecules that further consist of atoms. A compound may be any natural or non-natural material, for example, peptide or polypeptide sequences, organic or inorganic molecules or compositions, nucleic acid molecules, carbohydrates, lipids or combinations thereof. A compound generally refers to a chemical entity, whether in the solid, liquid or gaseous phase, and whether in a crude mixture or purified and isolated. Compounds encompass the chemical compound itself as well as, where applicable: amorphous and crystalline forms of the compound, including polymorphic forms, said forms in mixture or in isolation; free acid and free base forms of the compound; isomers of the compound, including geometric isomers, optical isomers, and tautomeric isomers, said optical isomers to include enantiomers and diastereomers, chiral isomers and non-chiral isomers, said optical isomers to include isolated optical isomers or mixtures of optical isomers including racemic and non-racemic mixtures; said geometric isomers to include transoid and cisoid forms, where an isomer may be in isolated form or in admixture with one or more other isomers; isotopes of the compound, including deuterium- and tritium-containing compounds, and including compounds containing radioisotopes, including therapeutically- and diagnostically-effective radioisotopes; multimeric forms of the compound, including dimeric, trimeric, etc. forms; salts of the compound, including acid addition salts and base addition salts, including organic counterions and inorganic counterions, and including zwitterionic forms, where if a compound is associated with two or more counterions, the two or more counterions may be the same or different; and solvates of the compound, including hemisolvates, monosolvates, disolvates, etc., including organic solvates and inorganic solvates, said inorganic solvates including hydrates; where if a compound is associated with two or more solvent molecules, the two or more solvent molecules may be the same or different.

In some embodiments, the term "compound" explicitly refers to small molecules. As used herein, the term "small molecule," as used herein, refers to a compound that is not itself the product of gene transcription or translation (*e.g*., protein, RNA, or DNA) and preferably has a low molecular weight, *e.g*., organic molecules having a molecular weight of less than about 2,000 daltons (Da). In some embodiments, small molecules have a molecular weight of less than about 1,500 Da. In other embodiments, small molecules have a molecular weight of less than about 1,000 Da. In still other embodiments, small molecules have a molecular weight of less than about 750 Da. In yet other embodiments, small molecules have a molecular weight of less than about 500 Da.

The phrase, "pharmaceutically acceptable prodrugs" as used herein, refers to derivatives of a compound, usually with significantly reduced pharmacological activity, which contain an additional moiety, which are susceptible to removal *in vivo* yielding the parent molecule as the pharmacologically active species. An example of a pro-drug is an ester, which is cleaved *in vivo* to yield a compound of interest. Pro-drugs of a variety of compounds, and materials and methods for derivatizing the parent compounds to create the pro-drugs, are known and may be adapted to the present invention.

As used herein the term "ester" refers to a group having the formula R'-COOR, where one of R' or R is, for example, an alkyl group, a haloalkyl group or an aromatic group, and the other of R' or R is the active moiety. A "pharmaceutically acceptable ester" refers to an ester which acts as a prodrug, *e.g*., an ester which is subject to at least partial removal *(e.g.,* by hydrolysis or other cleavage) from the core structure *in vivo.*

The term "protecting group" as used herein, refers to a particular functional moiety, *e.g*., O, S, or N, being temporarily blocked so that a reaction can be carried out selectively at another reactive site in a multifunctional compound. In preferred embodiments, a protecting group reacts selectively in good yield to give a protected substrate that is stable to the projected reactions; the protecting group must be selectively removed in good yield by readily available, preferably nontoxic reagents that do not attack the other functional groups; the protecting group forms an easily separable derivative (more preferably without the generation of new stereogenic centers); and the protecting group has a minimum of additional functionality to avoid further sites of reaction.

### Compounds of the Invention

In some embodiments, the compounds of the invention include compounds of formula (I): 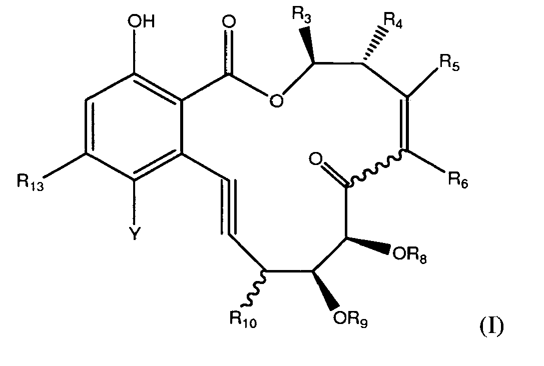 wherein
R₃ is a moiety selected from the group consisting of H and a C₁₋₆ alkyl group;
R₄ is a C₁₋₆ alkyl group, substituted with 0, 1, 2, or 3 halogen moieties;
R₅ is a hydrogen;
R₆ is a moiety selected from the group consisting of H and halogen; wherein R₅ and R₆ may be either cis or trans;
R₈ is H;
R₉ is a moiety selected from the group consisting of H and trifluoromethylcarbonyl; or R₈ and R₉ are taken together with the core structure to form a heterocyclydiyl of formula (a): 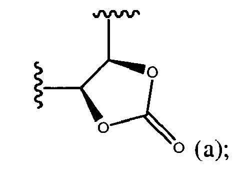
R₁₀ is a moiety selected from the group consisting of hydrogen and hydroxyl;
R₁₃ is a moiety selected from the group consisting of a C₁₋₆ alkylamino group and a C₁₋₆ alkoxy group, each of which is substituted with 0, 1, 2, or 3 hydroxyl moieties;
Y is a moiety selected from the group consisting of hydrogen, halogen and hydroxyl.

In some embodiments, R₃ is methyl. In some embodiments, R₄ is a methyl or a trifluoromethyl. In some embodiments, R₆ is an H or a fluorine. In some embodiments, R₅ and R₆ are cis. In other embodiments, R₅ and R₆ are trans. In some embodiments, Y is H, F, Cl or Br. In some embodiments, R₁₃ is an unsubstituted C₁₋₆ alkylamino, *e.g*., methylamino or ethylamino.

In some embodiments, the compound is at least one compound selected from the group consisting of: 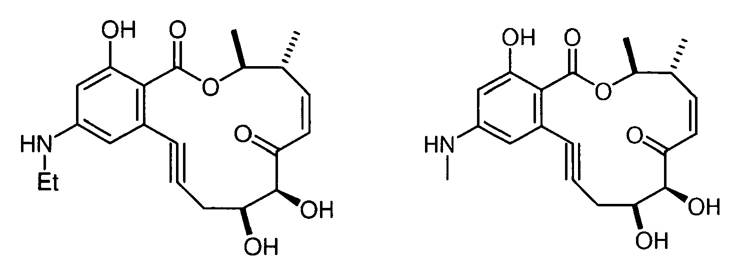 and pharmaceutically acceptable salts or prodrugs thereof.

The compounds of the present invention may include one or more asymmetric centers, and thus can exist in various isomeric forms, *e.g*., stereoisomers and/or diastereomers. Thus, compounds and pharmaceutical compositions of the present invention may be in the form of an individual enantiomer, diastereomer or geometric isomer, or may be in the form of a mixture of stereoisomers. In certain embodiments, the compounds of the invention are enantiopure compounds. In certain other embodiments, mixtures of stereoisomers or diastereomers are provided.

Compounds of the present invention may also have one or more double bonds that can exist as either the Z or E isomer, unless otherwise indicated. The invention additionally encompasses the compounds as individual isomers substantially free of other isomers and alternatively, as mixtures of various isomers, *e.g*., racemic mixtures of stereoisomers.

The compounds of the present invention may further exist as one or a combination of crystalline forms, *e.g*., polymorphs, solvates or hydrates of compound of formula (I). Various crystalline forms may be identified and/or prepared using different solvents, or different mixtures of solvents for recrystallization; by performing crystallizations at different temperatures; or by using various modes of cooling, ranging from very fast to very slow cooling during crystallizations. Different crystalline forms may also be obtained by heating or melting the compound followed by gradual or fast cooling. The presence of polymoiphs may be determined by solid probe NMR spectroscopy, IR spectroscopy, differential scanning calorimetry, powder X-ray diffractogram and/or other techniques.

### Synthetic Methodology

Guidance for preparing compounds useful for practicing the present invention can be found, for example, in WO 05/023792 on pages 32-38 and WO 03/076424 on pages 28-36, as well as in the Examples presented therein. The contents of these applications are incorporated herein in their entireties by this reference. These references in combination with the information contained herein and the additional body of knowledge with regard to macrolide chemistry provides a person of skill in the art with guidance on synthetic strategies, protecting groups, and other materials and methods useful for the synthesis of the compounds of the present invention. For example, the above references provide background information on preparing compounds similar to the compounds described herein or relevant intermediates, as well as information on formulation, uses, and administration of such compounds.

Specific guidance and examples are also provided herein relating to various exemplary compounds and intermediates thereof. Accordingly, compounds of the present invention and their preparation can be understood further by the examples herein, which illustrate some of the processes by which these compounds are prepared or used. It will be appreciated, however, that these examples do not limit the invention. Variations of the invention, now known or further developed, are considered to fall within the scope of the present invention as described herein and as hereinafter claimed.

According to the present invention, any available techniques can be used to make or prepare the inventive compounds or compositions including them. For example, a variety of solution phase synthetic methods such as those discussed in detail below may be used. Alternatively or additionally, the inventive compounds may be prepared using any of a variety combinatorial techniques, parallel synthesis and/or solid phase synthetic methods known in the art.

Starting materials and reagents useful in synthesizing the compounds of the present invention are either commonly available from commercial suppliers or can be prepared by methods known to a person of ordinary skill in the art. The starting materials, intermediates, and compounds of the present invention may be isolated and purified using conventional techniques, including filtration, distillation, crystallization, chromatography, and the like. They may be characterized using conventional methods, including physical constants and spectral data.

Exemplary syntheses of the compounds of the present invention are provided in the Exemplification. It will be appreciated that the methods as described herein can be applied to each of the compounds as disclosed herein and equivalents thereof. Additionally, the reagents and starting materials are well known to those skilled in the art. Although the schemes described in the above-cited references depict certain exemplary compounds, it will be appreciated that the use of alternate starting materials will yield other analogs of the invention.

Unless mentioned specifically, reaction mixtures were cooled to room temperature or below then quenched, when necessary, with either water or a saturated aqueous solution of ammonium chloride. Desired products were extracted by partitioning between water and a suitable water-immiscible solvent (*e.g*., ethyl acetate, dichloromethane, diethyl ether). The desired product containing extracts were washed appropriately with water followed by a saturated solution of brine. On occasions where the product containing extract was deemed to contain residual oxidants, the extract was washed with a 10% solution of sodium sulphite in saturated aqueous sodium bicarbonate solution, prior to the aforementioned washing procedure. On occasions where the product containing extract was deemed to contain residual acids, the extract was washed with saturated aqueous sodium bicarbonate solution, prior to the aforementioned washing procedure (except in those cases where the desired product itself had acidic character). On occasions where the product containing extract was deemed to contain residual bases, the extract was washed with 10% aqueous citric acid solution, prior to the aforementioned washing procedure (except in those cases where the desired product itself had basic character). Post washing, the desired product containing extracts were dried over anhydrous magnesium sulphate, and then filtered. The crude products were then isolated by removal of solvent(s) by rotary evaporation under reduced pressure, at an appropriate temperature (generally less than 45°C).

On occasions where triphenylphosphine oxide was a major byproduct of the reaction, the reaction mixture was added directly to a large volume of well-stirred hexane. The resultant precipitate of triphenylphosphine oxide was removed by filtration and the filtrate processed in the usual manner.

Unless mentioned specifically, chromatographic purification was used. Chromatographic purification refers to flash column chromatography on silica, using a single solvent or mixed solvent as eluent. Suitably purified desired product containing elutes were combined and concentrated under reduced pressure at an appropriate temperature (generally less than 45°C) to constant mass. If solid, final products were submitted for biological testing in their solid form. If freeze-drying was desired or necessary, final compounds were generally dissolved in 50% aqueous acetonitrile or other appropriate solvent or mixture of solvents, filtered and transferred to vials, then freeze-dried under high vacuum before submission for biological testing.

### Pharmaceutical Compositions

In another aspect of the present invention, pharmaceutical compositions are provided, which comprise any one of the compounds described herein (or a pharmaceutically acceptable salt or ester thereof), and optionally comprise a pharmaceutically acceptable carrier. In certain embodiments, these compositions optionally further comprise one or more additional therapeutic agents. Alternatively, a compound of this invention may be administered to a patient in need thereof in combination with the administration of one or more other therapeutic agents. For example, additional therapeutic agents for conjoint administration or inclusion in a pharmaceutical composition with a compound of this invention may be an anticancer agent approved for the treatment of cancer, as discussed in more detail herein, or it may be any one of a number of agents undergoing approval in the Food and Drug Administration that ultimately obtain approval for the treatment of cancer. It will also be appreciated that certain of the compounds of present invention can exist in free form for treatment, or where appropriate, as a pharmaceutically acceptable salt or ester thereof.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts of amines, carboxylic acids, and other types of compounds, are well known in the art. For example, S.M. Berge, *et al.* described pharmaceutically acceptable salts in detail in J. Pharmaceutical Sciences, 66: 1-19 (1977), incorporated herein by reference. The salts can be prepared *in situ* during the final isolation and purification of the compounds of the invention, or separately by reacting a free base or free acid function with a suitable reagent, as described generally below. For example, a free base function can be reacted with a suitable acid. Furthermore, where the compounds of the invention carry an acidic moiety, suitable pharmaceutically acceptable salts thereof may, include metal salts such as alkali metal salts, *e.g*. sodium or potassium salts; and alkaline earth metal salts, *e.g*. calcium or magnesium salts. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hernisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate.

Additionally, as used herein, the term "pharmaceutically acceptable ester" refers to esters that hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include formates, acetates, propionates, butyrates, acrylates and ethylsuccinates.

As described above, the pharmaceutical compositions of the present invention may optionally comprise a pharmaceutically acceptable carrier, which, as used herein, may include any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutical compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutical composition, its use is contemplated to be within the scope of this invention. Some examples of materials which can serve as pharmaceutically acceptable carriers include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil, sesame oil; olive oil; corn oil and soybean oil; glycols; such as propylene glycol; esters such as ethyl oleate and ethyl laurate; agar; buffering agents such as magnesium hydroxide and aluminum hydroxide; alginic acid; pyrogen free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Compositions of the present invention may be formulation to have any concentration desired. In some embodiments, the composition is formulated such that it comprises at least a therapeutically effective amount. In some embodiments, the composition is formulated such that it comprises an amount that would not cause one or more unwanted side effects. In certain embodiments, compositions are formulated so that the compound is present at a concentration of between about 1 mg/mL and about 20 mg/mL; between about 1 mg/mL and about 15 mg/mL; between about 1 mg/mL and about 10 mg/mL; between about 2 mg/mL and about 9 mg/mL; between about 3 mg/mL and about 8 mg/mL; between about 4 mg/mL and about 7 mg/mL; between about 4 mg/mL and about 6 mg/mL. In certain embodiments, compositions are formulated such that the compound is present at a concentration of about 5 mg/mL.

### Methods of Treatment

In some embodiments, based at least in part on their ability to inhibit the activity of MEK1, the compounds of the present invention are useful for treating and/or preventing a variety of cancers, as discussed in further detail herein. Exemplary cancers include solid tumors, leukemias, lymphomas and myelomas, such as multiple myeloma, B-cell lymphoma, chronic lymphocytic leukemia, acute myeloid leukemia, melanoma, ovarian cancer, thyroid cancer, pancreatic cancer, gliomas, such as astrocytomas, glioblastoma multiforme and other CNS tumors, colorectal cancer and/or breast cancer. Accordingly, in some aspects, the present invention is directed to the manufacture of a medicament for the treatment and/or prevention of cancer in a subject. For example, in some embodiments, the present invention is directed to treatment of cancer in a subject, wherein the treatment causes tumor cell regression. In other embodiments, the present invention is directed to the manufacture of a medicament for the treatment of a MEK1 associated cancer in a subject. In other embodiments, the present invention is directed to methods for the treatment of a NF-κB associated cancer in a subject. The methods of the present invention generally include administering an effective amount of a composition comprising a compound of the invention to the subject, such that the cancer is treated.

As used herein, the term "MEK1 associated cancer" refers to cancers that are associated with or otherwise affected by the activity or function of MEK1. Exemplary MEK1 associated cancers are discussed in more detail herein, and may include cancers associated with B-RAF mutations such as melanoma, ovarian cancer, thyroid cancer, colorectal cancer and/or breast cancer. As used herein, the term "NF-κB associated cancer" refers to cancers that are associated with or otherwise affected by the activity or function of NF-κB. Exemplary NF-κB associated cancers are discussed in more detail herein, and may include multiple myeloma and B-cell lymphoma.

Malignant transformation of normal to cancer cells typically requires the acquisition of several oncogenic traits such as uncontrolled cell division, resistance to programmed cell death (apoptosis), invasion and angiogenesis. These malignant transformation traits are believed to be the consequence of the accumulation of genetic alterations that result in dysregulated signal transduction circuits. Such genetic alterations can be point mutations that result in constitutive activation of key signal transducers such as Ras or the alterations can involve entire genes such as receptor tyrosine kinases epidermal growth factor receptor (EGFR) and ErbB2, which are overexpressed in many major human cancers including breast and lung tumors. Often such genetic alterations result in constitutive activation of common downstream signal transduction pathways such as the mitogen activated protein (MAP) kinase cascade: c-Raf-1, MEK1, Erk 1/2, p38 and JNK. These signal transduction pathways have been found constitutively activated in many human cancers and the hyperactivation of such pathways has been associated with poor prognosis and resistance to chemotherapy in cancer patients. Accordingly, in some embodiments, compounds of the present invention, which target MEK1, inhibit aberrant signal transduction pathways in tumor cells, which can in turn cause tumor regression by blocking the c-Raf-1/MEK1/Erk 1/2 pathway.

In some embodiments, compounds of the present invention include those which exhibit activity as inhibitors of MEK1. Accordingly, in some embodiments, an inhibitor of the present invention inhibits the activity of MEK1 by at least about 50% at a concentration of about 0.1 µM. In some embodiments, an inhibitor of the present invention inhibits the activity of MEK1 by at least about 51% at a concentration of about 0.1µM. In some embodiments, an inhibitor of the present invention inhibits the activity of MEK1 by at least about 52% at a concentration of about 0.1µM. In some embodiments, an inhibitor of the present invention inhibits the activity of MEK1 by at least about 53%, 54%, 55%, 56%, 57%, 58%, 59% or even 60% at a concentration of about 0.1µM. In further embodiments, an inhibitor of the present invention inhibits the activity of MEK1 by at least about 85% at a concentration of about 1.0µM. In still further embodiments, an inhibitor of the present invention inhibits the activity of MEK1 by at least about 86% at a concentration of about 1.0 µM. In other embodiments, an inhibitor of the present invention inhibits the activity of MEK1 by at least about 87%, 88%, 89%, 90%, 91%, 92%, 93%, 94%, or even 95% at a concentration of about 1.0 µM.

In some embodiments, the compounds of the present invention inhibit MEK1. For example, in some embodiments, compounds of the invention exhibit IC₅₀ values less than 10 µM. In certain other embodiments, compounds of the invention exhibit IC₅₀ values less than 7.5 µM. In certain embodiments, compounds of the invention exhibit IC₅₀ values less than 5 µM. In certain other embodiments, compounds of the invention exhibit IC₅₀ values less than 2.5 µM, less than 1 µM, less than 0.75 µM, less than 0.5 µM, less than 0.25 µM, less than 0.1 µM, less than 75 nM or less than 50 nM. All ranges and values between the listed values are meant to be encompassed by the present invention.

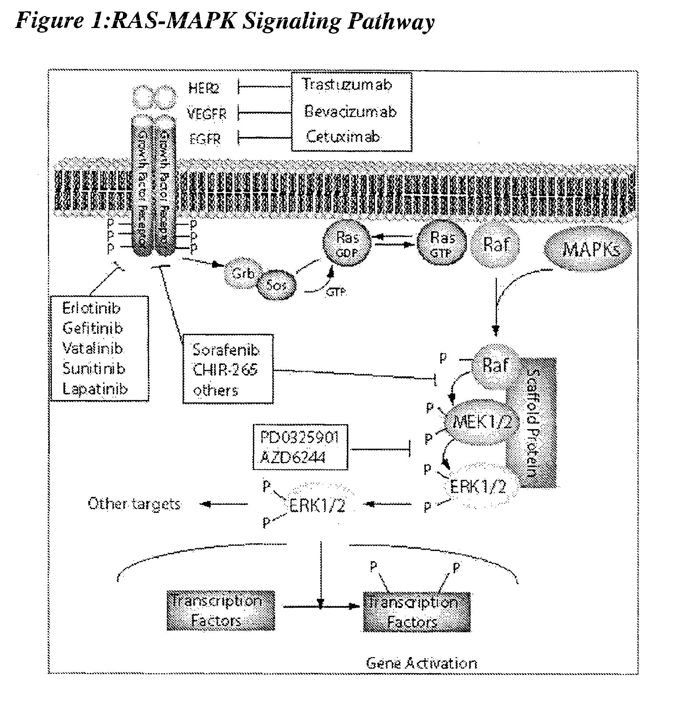

The RAS-MAPK signaling pathway is also viewed as an important pathway for anticancer therapies, based upon its central role in regulating the growth and survival of cells from a broad spectrum of human tumors (Figure 1). MEK1 is downstream of RAS and RAF proteins, which are often mutated and abnormally active in cancer. Tumors with elevated MEK signaling based on B-RAF mutations are thus attractive targets for using the compositions and methods of the present invention.

It is believed that the Ras/Raf/MEK/ERK signal transduction pathway regulates cell proliferation in diverse types of cells. Mutations in this pathway are often observed in transformed cell lines and frequently linked with human cancer. Davies et al. (Nature 417, 949-954, 2002), for example, identified B-RAF (an isoform of Raf) somatic missense mutations in 67% of malignant melanomas and 12% of colorectal cancers. All mutations were within the kinase domain with a single substitution, such as V600E (V599E), which accounts for 90% of these mutations. Mutated B-RAF proteins have elevated kinase activity, leading to constitutive activation of MEK, which then triggers ERK phosphorylation, and activates downstream pathway. It is believed that B-RAF mutated cancers, including melanoma, are attractive therapeutic targets for MEK inhibitors. The frequency of B-RAF mutations in melanoma and colorectal cancers, and the relative lack of effective therapies for advanced stages of these diseases suggest that inhibition of B-RAF activity may be an important new strategy in these cancer types. Moreover, in certain embodiments, B-RAF mutation can be used as molecular marker for "patient enrichment strategies" (*i..e.,* for targeting B-RAF mutation carrying patients that will benefit from compositions and methods of the invention).

In some embodiments, the compounds of the present invention are inhibitors of B-RAF mutated cancer cell growth, and thus may selectively target B-RAF mutated cancers. Accordingly, in some embodiments, the present invention provides compositions and methods for the treatment of B-RAF mutated cancers. Compounds of the present invention include compounds which exhibit an antiproliferative activity against B-RAF mutated cancers; exhibit an antiproliferative effect on suitable cell lines maintained *in vitro,* or in animal studies using a scientifically acceptable model; and/or exhibit a favorable therapeutic profile (*e.g*., safety, efficacy, and stability).

In certain embodiments, compounds of the invention are useful for treating and/or preventing tumors with elevated MEK signaling, including, but not limited to, those with B-RAF mutations. A number of proteins have received attention as targets for elevated MEK signaling (see Table 1 below, modified from Table 1 in Nature Reviews Cancer: 4:937-947, 2004). Mutations in these proteins could lead to activation of MEK-ERK pathway. Specifically, ovarian cancer, thyroid cancer, colorectal cancer and melanoma show high frequencies of B-RAF mutations.

**Table 1**

| Tumor type | Pathway mutations in patient tumors |
|---|---|
| Colon | KRAS (45%), B-RAF (12%) |
| Pancreatic | KRAS (90%) |
| Ovarian | B-RAF (30%) |
| Melanoma | NRAS(15%), B-RAF (67%) |
| Non-small-cell lung | KRAS (35%) |
| Papillary thyroid | HRAS, KRAS and NRAS (60%); |
| | B-RAF (30-70%) |
| ALL, AML | NRAS (30%) |

Accordingly, in certain embodiments, the methods of the present invention include methods for treating and/or preventing tumors with elevated MEK signaling, including, but not limited to, those with B-RAF mutations. In certain embodiments, compounds of the invention are useful for treating and/or preventing melanoma, colorectal cancer, ovarian cancer and/or thyroid cancer. Accordingly, in certain embodiments, the methods of the present invention include methods for treating and/or preventing a B-RAF mutated cancer. In certain embodiments, compounds of the invention are useful for treating and/or preventing cancers carrying a V600E mutation. In certain embodiments, the methods of the present invention include methods for treating and/or preventing a B-RAF mutated cancer carrying a V600E mutation.

It is also believed that, in some embodiments, the compounds of the present invention are useful in targeting hematological malignancies as well as solid tumors. In certain embodiments, compounds of the invention exhibit anticancer activity with evidence of apoptosis in B-cell non-Hodgkin's lymphoma, possibly by inhibition of MEKK1, an upstream molecule for NF-κB activation, which leads to chemoresistance to apoptosis. Accordingly, in certain embodiments, the methods of the present invention include methods for treating and/or preventing hematologic malignant cancer cell growth such as leukemias (*e.g*., chronic lymphocytic leukemia, acute myeloid leukemia), myelomas (*e.g*., multiple myeloma) and lymphomas (*e.g*., B-cell lymphomas).

In still other embodiments, the compounds of the present invention inhibit the activity of NF-κB. For example, in some embodiments, compounds of the invention exhibit IC₅₀ values less than 10 µM. In certain other embodiments, compounds of the invention exhibit IC₅₀ values less than 7.5 µM. In certain embodiments, compounds of the invention exhibit IC₅₀ values less than 5 µM. In certain other embodiments, compounds of the invention exhibit IC₅₀ values less than 2.5 µM, less than 1 µM, less than 0.75 µM, less than 0.5 µM, less than 0.25 µM, less than 0.1 µM, less than 75 nM, less than 50 nM, less than 25 nM, less than 10 nM, less than 7.5 nM or less than 5 nM. All ranges and values between the listed values are meant to be encompassed by the present invention.

In still other embodiments, the compounds of the present invention inhibit the growth of tumor cell lines *in vitro.* For example, in some embodiments, compounds of the invention exhibit IC₅₀ values less than 10 µM. In certain other embodiments, compounds of the invention exhibit IC₅₀ values less than 7.5 µM. In certain embodiments, compounds of the invention exhibit IC₅₀ values less than 5 µM. In certain other embodiments, compounds of the invention exhibit IC₅₀ values less than 2.5 µM, less than 1 µM, less than 0.75 µM, less than 0.5 µM, less than 0.25 µM, less than 0.1 µM, less than 75 nM or less than 50 nM. All ranges and values between the listed values are meant to be encompassed by the present invention.

In some embodiments, compounds of the invention cause tumor regression in *vivo, e.g.,* in suitable cancer cell xenograft models. In certain embodiments, compounds of the invention cause reduction of tumor size to below 70% of the size at the start of compound administration in a suitable cancer cell xenograft model. In certain embodiments, compounds of the invention cause reduction of tumor size to below 65% of the size at the start of compound administration in a suitable cancer cell xenograft model. In certain embodiments, compounds of the invention cause reduction of tumor size to below 60% below 55% or below 50% of the size at the start of compound administration in a suitable cancer cell xenograft model. All values and ranges between the listed values are intended to be encompassed by the present invention.

In certain embodiments, compounds of the invention cause inhibition of tumor growth *in vivo, e.g.,* in suitable cancer cell xenograft models. In certain embodiments, compounds of the invention cause inhibition of tumor growth in treated animals by > 50% compared to that of control animals (*i.e*., "treated" tumor size < 50% "control" tumor size; or T/C value < 50%) in suitable cancer cell xenograft models. In certain embodiments, compounds of the invention have T/C values < 70%. In certain embodiments, compounds of the invention have T/C values < 65%, < 60%, or < 55%. All values and ranges between the listed values are intended to be encompassed by the present invention.

In certain embodiments, compounds of the invention exhibit inhibitory activity against NF-κB. As such, they are useful for treating and/or preventing cancers related to NF-κB activity. In certain embodiments, compounds of the invention are useful for treating and/or preventing hematologic malignancies such as multiple myeloma and/or B cell lymphomas. In some embodiments, compounds of the invention are beneficial for cancer patients showing resistance in conventional chemotherapy via NF-κB activation.

According to the present invention, the inventive compounds may be assayed in any of the available assays known in the art for identifying compounds having, for example, MEK1 inhibitory activity, NF-κB activation inhibitory activity and cancer cell growth inhibitory activity. For example, the assay may be cellular or non-cellular, *in vivo* or *in vitro,* high- or low-throughput format, etc.

### Dosages and Modes of Administration

It will be appreciated that the compounds and compositions, according to the method of the present invention, may be administered using any amount and any route of administration effective for the treatment of cancer. Thus, the expression "effective amount" as used herein, refers to a sufficient amount of agent to inhibit the growth of tumor cells, or refers to a sufficient amount to reduce the effects of cancer. The exact amount required will vary from subject to subject, depending on the species, age, and general condition of the subject, the severity of the diseases, the particular anticancer agent, its mode of administration, and the like. The compounds of the invention are preferably formulated in dosage unit form for ease of administration and uniformity of dosage. The expression "dosage unit form" as used herein refers to a physically discrete unit of therapeutic agent appropriate for the patient to be treated. It will be understood, however, that the total daily usage of the compounds and compositions of the present invention will be decided by the attending physician within the scope of sound medical judgment. The specific therapeutically effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts (see, for example, Goodman and Gilman's, "The Pharmacological Basis of Therapeutics", Tenth Edition, A. Gilman, J.Hardman and L. Limbird, eds., McGraw-Hill Press, 155-173, 2001, which is incorporated herein by reference in its entirety).

In certain embodiments, the compounds or compositions are administered systemically. As used herein, "systemic administration" refers to any means by which a compound of the invention can be made systemically available. For example, systemic administration encompasses enteral (*e.g*., oral and rectal) and parenteral methods of administration. In certain embodiments, systemic administration encompasses intravenous administration, intraperitoneal administration, intramuscular administration, intracoronary administration, intraarterial administration (*e.g*., into a carotid artery), intradermal administration, subcutaneous administration, transdermal delivery, intratracheal administration, subcutaneous administration, intraarticular administration, intraventricular administration, inhalation (*e.g*., aerosol), intracerebral, nasal, naval, oral, intraocular, pulmonary administration, impregnation of a catheter, by suppository and direct injection into a tissue, or systemically absorbed topical or mucosal administration. Mucosal administration includes administration to the respiratory tissue, *e.g*., by inhalation, nasal drops, ocular drop, etc.; anal or vaginal routes of administration, *e.g*., by suppositories; and the like. In certain exemplary embodiments, the compositions of the invention are administered intravenously.

It will be appreciated that the inventive compound may be administered systemically in dosage forms, formulations or *e.g*. suitable delivery devices or implants containing conventional, non-toxic pharmaceutically acceptable carriers and adjuvants such that the compound effectiveness is optimized. For example, the inventive compound may be formulated together with appropriate excipients into a pharmaceutical composition, which, upon administration of the composition to the subject, systemically releases the active substance in a controlled manner. Alternatively, or additionally, compound dosage form designs may be optimized so as to increase the compound effectiveness upon administration. The above strategies (*i.e*., dosage form design and rate control of drug input), when used alone or in combination, can result in a significant increase in compound effectiveness and are considered part of the invention.

Furthermore, after formulation with an appropriate pharmaceutically acceptable carrier in a desired dosage, the pharmaceutical compositions of this invention can be administered to humans and other animals orally, rectally, parenterally, intracisternally, intravaginally, intraperitoneally, topically (as by powders, ointments, creams or drops), bucally, as an oral or nasal spray, or the like,. In certain embodiments, the compounds of the invention may be administered at dosage levels of about 0.001 mg/kg to about 50 mg/kg, from about 0.01 mg/kg to about 25 mg/kg, or from about 0.1 mg/kg to about 10 mg/kg of subject body weight per day, one or more times a day, to obtain the desired therapeutic effect. It will also be appreciated that dosages smaller than 0.001 mg/kg or greater than 50 mg/kg (for example 50-100 mg/kg) can be administered to a subject. In certain embodiments, compounds are administered orally or parenterally. In certain embodiments, administration is conducted by intermittent dosing one to three times a week.

In certain embodiments, the compounds of the invention may be administered at dosage levels of about 1-500 mg/m²; *e.g*., about 5-400 mg/m²; about 10-250 mg/m² one or more times a week to obtain the desired therapeutic effect. Specific ranges and values which fall between the listed ranges are also contemplated by the present invention.

In certain embodiments, the compounds of the invention may be administered intravenously one to five times a week. In certain embodiments, the compounds of the invention may be administered intravenously once a week. In certain embodiments, the compounds of the invention may be administered intravenously twice a week. In certain embodiments, the compounds of the invention may be administered intravenously three times a week.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable reparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension or crystalline or amorphous material with poor water solubility. The rate of absorption of the drug then depends upon its rate of dissolution that, in turn, may depend upon crystal size and crystalline form. Alternatively, delayed absorption of a parenterally administered drug form is accomplished by dissolving or suspending the drug in an oil vehicle. Injectable depot forms are made by foaming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include (poly(orthoesters) and poly(anhydrides). Depot injectable formulations are also prepared by entrapping the drug in liposomes or microemulsions, which are compatible with body tissues.

Compositions for rectal or vaginal administration are preferably suppositories which can be prepared by mixing the compounds of this invention with suitable nonirritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose and starch. Such dosage forms may also comprise, as in normal practice, additional substances other than inert diluents, *e.g*., tableting lubricants and other tableting aids such as magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions, which can be used, include polymeric substances and waxes.

The present invention encompasses pharmaceutically acceptable topical formulations of inventive compounds. The term "pharmaceutically acceptable topical formulation", as used herein, means any formulation which is pharmaceutically acceptable for intradermal administration of a compound of the invention by application of the formulation to the epidermis. In certain embodiments of the invention, the topical formulation comprises a carrier system. Pharmaceutically effective carriers include, but are not limited to, solvents (*e.g*., alcohols, poly alcohols, water), creams, lotions, ointments, oils, plasters, liposomes, powders, emulsions, microemulsions, and buffered solutions (*e.g*., hypotonic or buffered saline) or any other carrier known in the art for topically administering pharmaceuticals. A more complete listing of art-known carriers is provided by reference texts that are standard in the art, for example, Remington's Pharmaceutical Sciences, 16th Edition, 1980 and 17th Edition, 1985, both published by Mack Publishing Company, Easton, Pa., the disclosures of which are incorporated herein by reference in their entireties. In certain other embodiments, the topical formulations of the invention may comprise excipients. Any pharmaceutically acceptable excipient known in the art may be used to prepare the inventive pharmaceutically acceptable topical formulations. Examples of excipients that can be included in the topical formulations of the invention include, but are not limited to, preservatives, antioxidants, moisturizers, emollients, buffering agents, solubilizing agents, other penetration agents, skin protectants, surfactants, and propellants, and/or additional therapeutic agents used in combination to the inventive compound. Suitable preservatives include, but are not limited to, alcohols, quaternary amines, organic acids, parabens, and phenols. Suitable antioxidants include, but are not limited to, ascorbic acid and its esters, sodium bisulfite, butylated hydroxytoluene, butylated hydroxyanisole, tocopherols, and chelating agents like EDTA and citric acid. Suitable moisturizers include, but are not limited to, glycerine, sorbitol, polyethylene glycols, urea, and propylene glycol. Suitable buffering agents for use with the invention include, but are not limited to, citric, hydrochloric, and lactic acid buffers. Suitable solubilizing agents include, but are not limited to, quaternary ammonium chlorides, cyclodextrins, benzyl benzoate, lecithin, and polysorbates. Suitable skin protectants that can be used in the topical formulations of the invention include, but are not limited to, vitamin E oil, allatoin, dimethicone, glycerin, petrolatum, and zinc oxide.

In certain embodiments, the pharmaceutically acceptable topical formulations of the invention comprise at least a compound of the invention and a penetration enhancing agent. The choice of topical formulation will depend or several factors, including the condition to be treated, the physicochemical characteristics of the inventive compound and other excipients present, their stability in the formulation, available manufacturing equipment, and costs constraints. As used herein the term " penetration enhancing agent " means an agent capable of transporting a pharmacologically active compound through the stratum corneum and into the epidermis or dermis, preferably, with little or no systemic absorption. A wide variety of compounds have been evaluated as to their effectiveness in enhancing the rate of penetration of drugs through the skin. See, for example, Percutaneous Penetration Enhancers, Maibach H. I. and Smith H. E. (eds.), CRC Press, Inc., Boca Raton, Fla. (1995), which surveys the use and testing of various skin penetration enhancers, and Buyuktimkin et al., Chemical Means of Transdermal Drug Permeation Enhancement in Transdermal and Topical Drug Delivery Systems, Gosh T. K., Pfister W. R., Yum S. I. (Eds.), Interpharm Press Inc., Buffalo Grove, III. (1997). In certain exemplary embodiments, penetration agents for use with the invention include, but are not limited to, triglycerides (e.g., soybean oil), aloe compositions (*e.g*., aloe-vera gel), ethyl alcohol, isopropyl alcohol, octolyphenylpolyethylene glycol, oleic acid, polyethylene glycol 400, propylene glycol, N-decylmethylsulfoxide, fatty acid esters (*e.g*., isopropyl myristate, methyl laurate, glycerol monooleate, and propylene glycol monooleate) and N-methyl pyrrolidone.

In certain embodiments, the compositions may be in the form of ointments, pastes, creams, lotions, gels, powders, solutions, sprays, inhalants or patches. In certain exemplary embodiments, formulations of the compositions according to the invention are creams, which may further contain saturated or unsaturated fatty acids such as stearic acid, palmitic acid, oleic acid, palmito-oleic acid, cetyl or oleyl alcohols, stearic acid being particularly preferred. Creams of the invention may also contain a non-ionic surfactant, for example, polyoxy-40-stearate. In certain embodiments, the active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a compound to the body. Such dosage forms are made by dissolving or dispensing the compound in the proper medium. As discussed above, penetration enhancing agents can also be used to increase the flux of the compound across the skin. The rate can be controlled by either providing a rate controlling membrane or by dispersing the compound in a polymer matrix or gel.

In certain exemplary embodiments, pharmaceutically acceptable topical formulations of the invention are contained in a patch that is applied adjacent to the area of skin to be treated. As used herein a "patch" comprises at least a topical formulation and a covering layer, such that, the patch can be placed over an area of skin. Any patch known in the art can be used in conjunction with the compositions and methods of the present invention.

In certain exemplary embodiments, the inventive compounds may be used as coating for stents. Guidance for using compounds of the invention in this capacity can be found, for example, in WO 05/023792.

### Coadministration

It will also be appreciated that the compounds and pharmaceutical compositions of the present invention can be formulated and employed in combination therapies, that is, the compounds and pharmaceutical compositions can be formulated with or administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another anticancer agent), or they may achieve different effects (*e.g*., control of any adverse effects).

For example, other therapies or anticancer agents that may be used in combination with the inventive compounds of the present invention include surgery, radiotherapy (in but a few examples, γ-radiation, neutron beam radiotherapy, electron beam radiotherapy, proton therapy, brachytherapy, and systemic radioactive isotopes, to name a few), endocrine therapy, biologic response modifiers (interferons, interleukins, and tumor necrosis factor (TNF) to name a few), hyperthermia and cryotherapy, agents to attenuate any adverse effects (*e.g*., antiemetics), and other approved chemotherapeutic drugs, including, but not limited to, alkylating drugs (mechlorethamine, chlorambucil, Cyclophosphamide, Melphalan, Ifosfamide), antimetabolites (Methotrexate), purine antagonists and pyrimidine antagonists (6-Mercaptopurine, 5-Fluorouracil, Cytarabile, Gemcitabine), spindle poisons (Vinblastine, Vincristine, Vinorelbine, Paclitaxel), podophyllotoxins (Etoposide, Irinotecan, Topotecan), antibiotics (Doxorubicin, Bleomycin, Mitomycin), nitrosoureas (Carmustine, Lomustine), inorganic ions (Cisplatin, Carboplatin), enzymes (Asparaginase), and hormones (Tamoxifen, Leuprolide, Flutamide, and Megestrol), to name a few. Other anticancer agents that may be used in combination with compounds or compositions of the present invention, include a therapeutic antibody or antibody fragment, such as a human, humanized, chimeric, and/or single-chain antibody or antibody fragment (*e.g*., Fv, Fab, Fab', F(ab')_{2,} dAb) or the like (*e.g*., Trastuzumab, Bevacizumab, Cetuximab, Rituximab). For a more comprehensive discussion of updated cancer therapies see, The Merck Manual, Seventeenth Ed. 1999, the entire contents of which are hereby incorporated by reference. See also the National Cancer Institute (CNI) website (www.nci.nih.gov) and the Food and Drug Administration (FDA) website for a list of the FDA approved oncology drugs (www.fda.gov/cder/cancer/druglistframe - See Appendix A).

In certain embodiments, compounds of the invention exhibit synergy in combination therapy with another anticancer agent. In certain exemplary embodiments, compounds of the invention exhibit synergy in combination therapy with SN-38 (active metabolite of CPT-11). In some embodiments, compounds of the invention are more effective in tumor cell killing when used in combination with another anticancer agent (*e.g*., SN-38 (active metabolite of CPT-11)).

In certain embodiments, the pharmaceutical compositions of the present invention further comprise one or more additional therapeutically active ingredients (*e.g*., chemotherapeutic and/or palliative). For purposes of the invention, the term "*Palliative*" refers to treatment that is focused on the relief of symptoms of a disease and/or side effects of a therapeutic regimen, but is not curative. For example, palliative treatment encompasses painkillers, antinausea medications and anti-sickness drugs. In addition, chemotherapy, radiotherapy and surgery can all be used palliatively (that is, to reduce symptoms without going for cure; *e.g*., for shrinking tumors and reducing pressure, bleeding, pain and other symptoms of cancer).

### Treatment Kits

In some aspects, the present invention provides a kit for carrying out the methods in accordance with the present invention. In general, the pharmaceutical pack or kit comprises one or more of the ingredients of the pharmaceutical compositions of the invention. For example, the kit may include one or more containers filled (completely or partially) with a compound or composition of the present invention. Such kits are suited for, *e.g*., the delivery of solid oral forms such as tablets or capsules. In some embodiments, the kit includes a number of unit dosages, and may also include a card having the dosages oriented in the order of their intended use. If desired, a memory aid can be provided, for example in the form of numbers, letters, or other markings or with a calendar insert, designating the days in the treatment schedule in which the dosages can be administered. Alternatively, placebo dosages, or calcium dietary supplements, either in a form similar to or distinct from the dosages of the pharmaceutical compositions, can be included to provide a kit in which a dosage is taken every day. In some embodiments, the kit includes instructions or directions for the use of the other components of the kit. In some embodiments, the kit comprises a composition of the present invention and a second therapeutic agent, with instructions on coadministration of the two agents. Optionally associated with such container(s) can be a notice in the form prescribed by a governmental agency regulating the manufacture, use or sale of pharmaceutical products, which notice reflects approval by the agency of manufacture, use or sale for human administration.

### EQUIVALENTS

Those skilled in the art will recognize, or be able to ascertain using no more than routine experimentation, many equivalents to the specific embodiments of the invention described herein. Such equivalents are intended to be encompassed by the following claims.

### INCORPORATION BY REFERENCE

The contents of all references, patents, and patent applications cited throughout this application are hereby incorporated by reference.

### EXEMPLIFICATION

The compounds of this invention and their preparation can be understood further by the examples that illustrate some of the processes by which these compounds are prepared or used. It will be appreciated, however, that these examples do not limit the invention. Variations of the invention, now known or further developed, are considered to fall within the scope of the present invention as described herein and as hereinafter claimed.

The schemes listed herein are merely illustrative of some methods by which the compounds of this invention can be synthesized, and it is to be understood that various modifications to these schemes can be made.

### Example 1: Synthesis of Compounds

### General Reaction Procedures:

Unless mentioned specifically, reaction mixtures were stirred using a magnetically driven stirrer bar. An inert atmosphere generally refers to either dry argon or dry nitrogen. Reactions were monitored either by thin layer chromatography (TLC), mass spectrometry (MS), proton nuclear magnetic resonance (NMR) or high-pressure liquid chromatography (HPLC), of a suitable sample of the reaction mixture.

Common abbreviations include m-CPBA: meta-chloroperbenzoic acid; DDQ: 2,3-dichloro-5,6-dicyano-1,4-benzoquinone; DEAD: diethyl azodicarboxylate; DIBAL-H: diisobutyl aluminum hydride; DMAP: N,N-dimethylaminopyridine; DMF: N,N-dimethylformamide; HMPA: hexamethylphosphoramide; LDA: lithium diisopropyl amide; LiHMDS: lithium bis (trimethylsilyl) amide; PCC: pyridinium chlorochromate; TBAF: tetrabutylammonium fluoride; THF: tetrahydrofuran; CH₂Cl₂ or DCM: methylene chloride; MPM: para-methoxy benzyl; Boc: tert-butyl oxy carbamate; TfOH: trifluoromethane sulfonic acid; Tf2O: trifluoromethane sulfonic acid anhydride; TFA: trifluoroacetic acid; and TFAA: trifluoroacetic acid anhydride.

### Synthesis of a common starting material

### Compound 003:

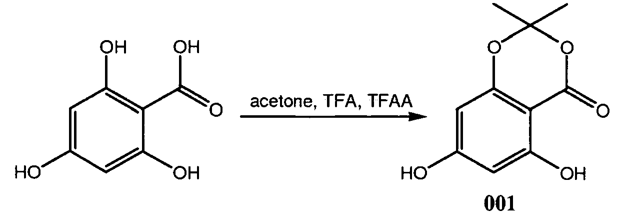

To a solution of trihydroxy-benzoic acid (120 g) in 350 mL of acetone, 500 mL of TFA (trifluoro acetic acid) was added at 40°C under stirring. After 1h at 40°C, 300 mL of TFAA (trifluoro acetic anhydride) was added. The mixture was heated for 3 days. The mixture was distilled under house vacuum at 50°C to remove solvents. The crude product was then diluted with 4L of CH₂Cl₂, washed with water, sat. NaHCO₃, dried and concentrated to give 85 g of semi pure solid **001**. The solid was crystallized in EtOH (1g/ 2mL) to give 20g of pure crystal. The mother liquor was then purified by silica gel with CH₂CL₂ to 5% MeOH/CH₂Cl₂ to give 55g of additional product **001**.

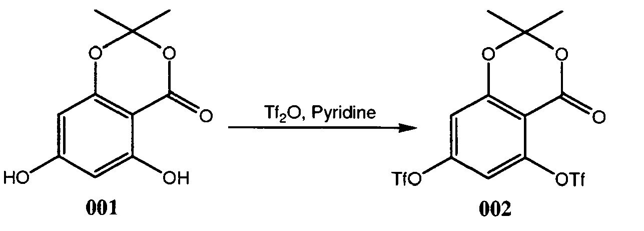

To a solution of **001** (50g, 238 mmol) in 156 mL of pyridine, Tf₂O (100mL, 595 mmol, 2.5eq.) was added at 0°C in 3h. Then it was warmed to room temperature and stirred for 2h. The reaction mixture was diluted with water. The mixture was filtered, and the solid on the filter was washed with water, dried under vacuum to give 100g of solid **002**.

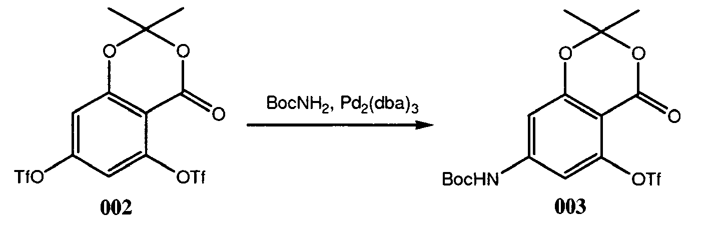

Ditriflate **002** (45.35g), BocNH₂ (17.22g), Pd_{2µ}(dba)₃ (4.38g) and P*t*-Bu₃ (4.38g) were mixed in 150 mL of toluene. Tri-ethylamine (26.92 mL) was added to this mixture and the reaction was heated under inert atmosphere at 80°C for 4h. The crude reaction mixture was cooled and filtered through a pad of celite. The filtrates were concentrated and purified on silica gel with hexane/EtOAc, 9:1, 4:1 to give 28.3 g of desired product, compound **003**.

### Synthesis of selected Compounds of the Invention

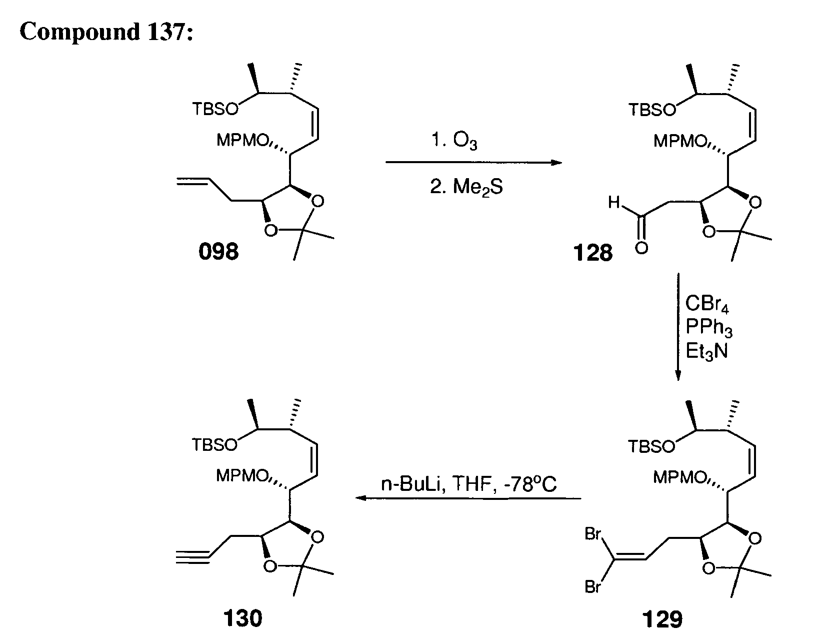

To a solution of compound **098** (1.0g, 1.98mmol) in MeOH/CH₂Cl₂ (5ml/5ml) at -78°C was bubbled O₃. After 7min, N₂ was bubbled through for 10min and 2 pipettes of Me₂S were added. The reaction mixture was warmed up to room temperature and solvents were removed by evaporation. Purification by silica gel chromatography gave compound **128** (735mg, 73%).

To a reaction flask containing CBr₄ (969mg, 2.90mmol) and PPh₃ (15.2g 5.80mmol) in 10ml of CH₂Cl₂ was added a solution of compound **128** (734mg, 1.45mmol) and Et₃N (202µl, 1.45mmol) in 5ml of CH₂Cl₂ at 0°C. The ice bath was removed and the reaction mixture was stirred at room temperature for 1 hour. After titration with Hexane, purification by silica gel chromatography gave compound **129** (557g, 58%).

To a solution of compound **129** (557mg, 0.84mmol) in THF (5ml) at -78°C was added n-BuLi (840µl, 2.5M in Hexane). After 1 hour the reaction was quenched with saturated aqueous NH₄Cl. The aqueous layer was extracted with MTBE. Purification by silica gel chromatography gave compound **130** (360mg, 85%).

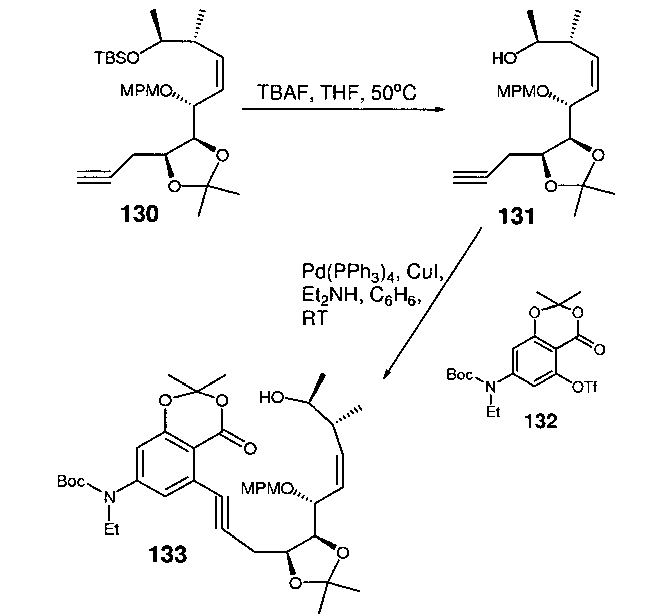

To a solution of compound **130** (358mg, 0.71mmol) in THF (5ml) at room temperature was added TBAF (1.1ml, 1.0M in THF). The reaction mixture was heated at 50°C for 4 hours. The mixture was then diluted with MTBE, washed with water and brine. Purification by silica gel chromatography gave compound **131** (277mg, 100%).

To a solution of compound **131** (263.8mg, 0.678mmol) and compound **132** (637.4mg, 1.356mmol) in benzene (1.8ml) and ET₂NH (1.2ml) at room temperature was added Pd(PPh₃)₄ (39.2mg, 0.034mmol) and Cul (12.9mg, 0.068mmol). The reaction mixture was stirred for 5 hours. The mixture was then diluted with MTBE, washed with saturated NH₄Cl, saturated NaHCO₃ and brine. Purification by silica gel chromatography gave compound **133** (251.5mg, 52%).

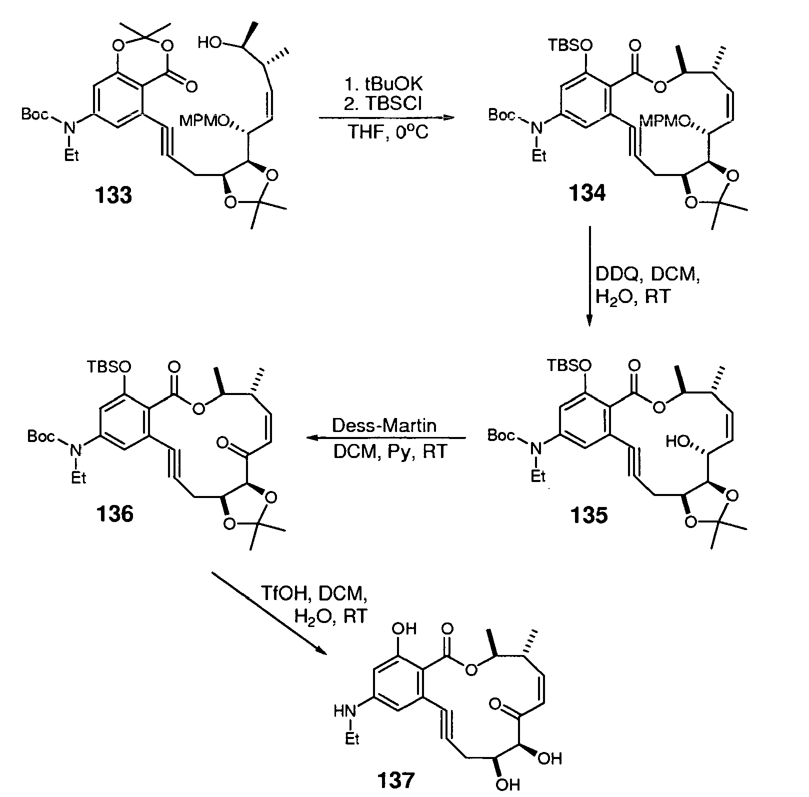

To a solution of t-BuOK (565µl, 1.0M in THF) in THF (13.0ml) at 0°C was added the solution of compound **133** (200.0mg, 0.28mmol) in THF (8.0ml) at rate of 0.1ml/min After 1 hour, TBSCI (169mg, 0.56mmol) was added. After 5minutes, the reaction mixture was quenched with saturated NaHCO₃, diluted with water and extracted with MTBE. Purification by silica gel chromatography gave compound **134** (175.4mg, 82%).

To a solution of compound **134** (200.0mg, 0.28mmol) in DCM 4.0ml) and water (1.0ml) at room temperature was added DDQ (96.2mg, 0.42mmol). After 2 hours, the reaction was quenched with 10% Na₂S₂O₃ in a saturated NaHCO₃ aqueous solution and extracted with MTBE. Purification by silica gel chromatography gave compound **135** (133.1mg, 74%).

To a solution of compound **135** (133.0mg, 0.207mmol) in DCM (3.0ml) at room temperature was added pyridine (42.0µl, 0.518mmol) and Dess-Martin reagent (175.0mg, 0.414mmol). After 1 hour, the reaction was quenched with 10% Na₂S₂O₃ in saturated NaHCO₃ aqueous solution and extracted with DCM. Purification by silica gel chromatography gave compound **136** (114.8mg, 86%).

To a solution of compound **136** (1 13.0mg, 0.176mmol) in DCM (2.0ml) at 0°C was added water (19.0µl, 1.056mmol) and TfOH (46.0µl, 0.528mmol). The reaction was allowed to warm up to room temperature. After 30 minutes, the reaction was quenched with saturated NaHCO₃ and extracted with DCM. Purification by silica gel chromatography gave compound **137** (46.4mg, 68%). ¹H NMR (400 MHz, CDCl₃) δ 1.20 (d, J=6.8Hz, 3H), 1.25 (t, J=6.8, 3H), 1.38 (d, J=6.0, 3H), 2.47-2.48 (m, 2H), 2.85 (d, J=11.2Hz), 3.70-3.21 (m, 2H), 3.41-3.45 (m, 1H), 3.95 (d, J=5.2, 1H), 4.00-4.40 (m, 1H), 4.27-4.33 (m, 1H), 4.54-4.56 (m, 1H), 4.93-5.00 (m, 1H), 6.04 (d, J=2.4, 1H), 6.23-6.32 (m, 3H), 12.10 (s, 1H).

### Compound 144:

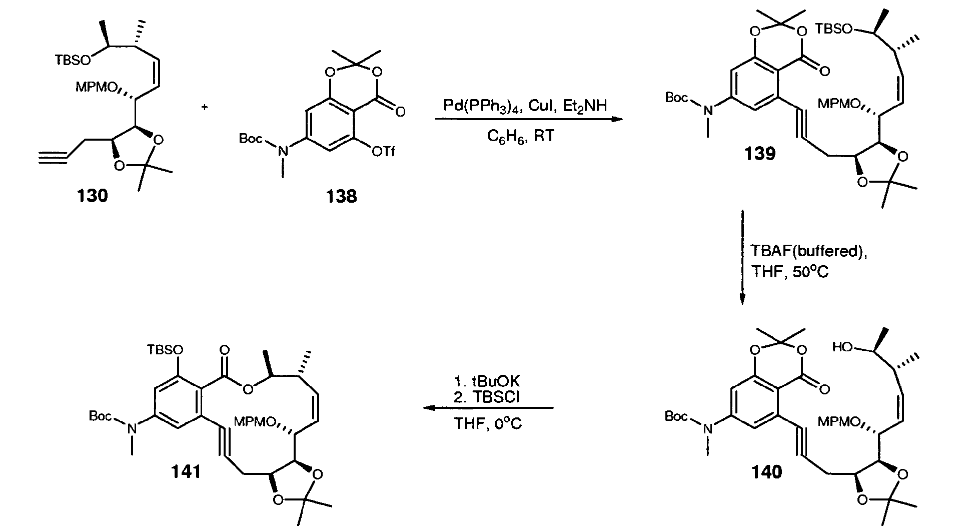

To a solution of compound **130** (475mg, 0.944mmol) and compound **138** (558mg, 1.227mmol) in benzene (2.2ml) and Et₂NH (1.5ml) at room temperature was added Pd(PPh₃)₄ (55mg, 0.0472mmol) and CuI (18mg, 0.0944mmol). The reaction mixture was stirred for 3 hours. The reaction mixture was then diluted with MTBE, washed with saturated NH₄Cl, saturated NaHCO₃ and brine. Purification by silica gel chromatography gave compound **139** (485mg, 56%).

To a solution of compound **139** (485mg, 0.60mmol) in THF (5.0ml) was added buffered TBAF (3.6ml, 0.5N buffered with imidazole/HCI) at room temperature. The reaction mixture was heated at 50°C for 60 hours. The reaction mixture was then diluted with MTBE and washed with water and brine. Purification by silica gel chromatography gave compound **140** (280.8mg, 67%).

To a solution of t-BuOK (720µl, 1.0M in THF) in THF (13.5ml) at 0°C was added the solution of compound **140** (250mg, 0.36mmol) in THF (13.5ml). After 2 hours, TBSC1 (217mg, 1.44mmol) was added. After 5 minutes, the reaction mixture was quenched with saturated NaHCO₃ diluted with water and extracted with MTBE. Purification by silica gel chromatography gave compound **141** (155mg, 52%).

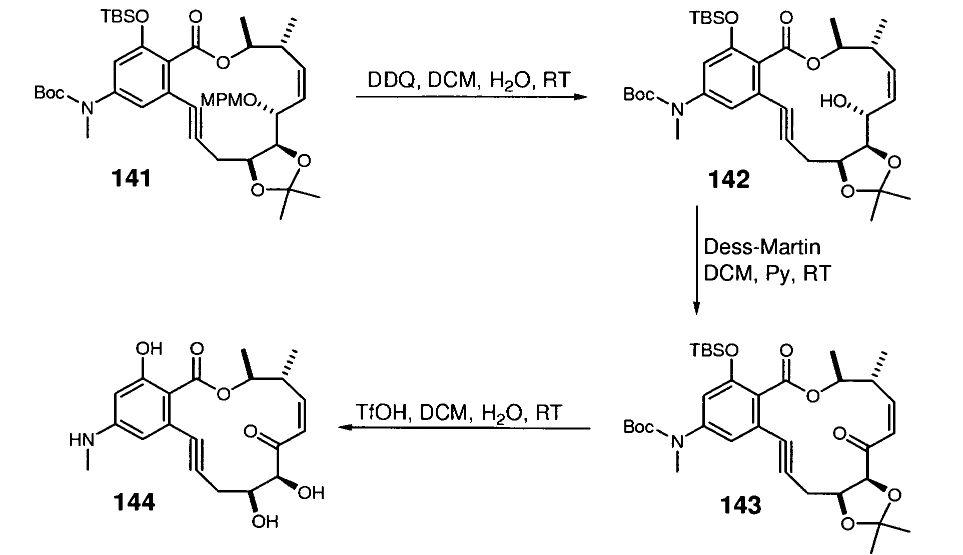

To a solution of compound **141** (153mg, 0.204mmol) in DCM 4.0ml) and water (1.0ml) at room temperature was added DDQ (69.5mg, 0.306mmol). After 2.5 hours, the reaction was quenched with saturated NaHCO₃ and extracted with MTBE. Purification by silica gel chromatography gave compound **142** (124.0mg, 96%).

To a solution of compound **142** (124.0mg, 0.197mmol) in DCM (5.0ml) at room temperature was added pyridine (40.0µl, 0.492mmol) and Dess-Martin reagent (167.0mg, 0.394mmol). After 1hr, the reaction was quenched with 10% Na₂S₂O₃ in saturated NaHCO₃ aqueous solution and extracted with DCM. Purification by silica gel chromatography gave compound **143** (122.8mg, 99%).

To a solution of compound **143** (54.5mg, 0.087mmol) in DCM (2.0ml) at 0°C was added water (9.4µl, 0.522mmol) and TfOH (23.0µl, 0.261mmol). The reaction was allowed to warm up to room temperature. After 30 minutes, it was quenched with saturated NaHCO₃ and extracted with DCM. Purification by silica gel chromatography gave compound **144** (30.0mg, 92%). ¹H NMR (400 MHz, CDCl₃) 1.20 (d, J=6.8Hz, 3H), 1.38 (d, J=6.0Hz, 3H), 2.47-2.48(m, 2H), 2.85 (d, J=5.2Hz 3H), 3.40-3.50 (m, 1H), 3.94 (d, J=5.2), 4.12-4.19 (m, 1H), 4.28-4.33 (m, 1H), 4.54-4.56 (m, 1H), 4.93-5.00 (m, 1H), 6.05 (d, J=2.4Hz, 1H), 6.30 (s, 1H), 6.33 (m, 2H), 12.11 (s, 1H).

### Example 2

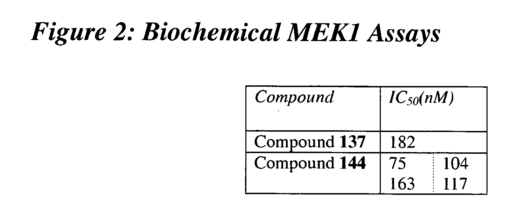

A biochemical MEK1 assay was carried out to investigate whether compounds of the invention are MEK1 inhibitors. Biochemical MEK1 inhibition of the compounds was assessed using an ELISA assay. All assays were performed in a 96 well format. A plate coated with ERK2, a substrate protein for MEK1 was used for biochemical MEK1 assay. In a final volume of 40 µL, MEK1 (25 ng) was incubated with 50 mmol/L HEPES, 20 mmol/L MgCl₂, 4 mmol/L MnCl₂, and 0.5 mmol/L Na₃VO₄ in the absence or presence of the test compounds. The reaction was initiated by the addition of 1µmol/L ATP. After incubation for 40 minutes at 30°C, the reaction mixture was discarded and the plate was then washed 4 times with 0.05% Tween20/PBS. Each well was incubated with 100 µL of 1:4000 diluted phospho-ERK1/2 monoclonal antibody (Cell Signaling, MA) for 1.5 hours at room temperature. The antibody was then discarded and the plate was washed 6 times with 0.05% Tween20/PBS. Finally, 100 µL of 1:2000 diluted of HRP conjugated anti-mouse IgG antibody (Cell Signaling, MA) was added. After incubation for 90 minutes, this antibody was discarded and the plate was washed 8 times with 0.05% Tween20/PBS. Phosphorylated ERK2 was then visualized by peroxidase reaction, and read at 450 nm. Figure 2 is a table depicting results from MEK1 assays. These results show that compounds of the invention are potent MEK1 inhibitors with low nM activity.

## Claims

1. A compound of formula (I) 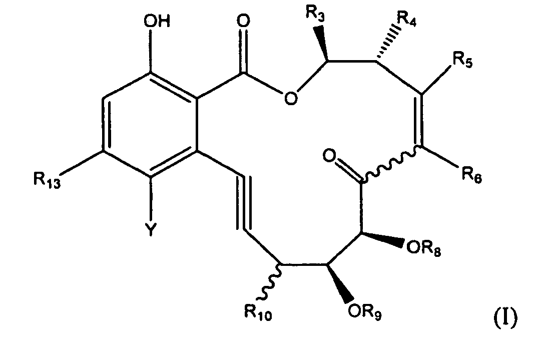 wherein
R₃ is a moiety selected from the group consisting of H and a C₁₋₆ alkyl group;
R₄ is a C₁₋₆ alkyl group, substituted with 0, 1, 2, or 3 halogen moieties;
R₅ is a hydrogen;
R₆ is a moiety selected from the group consisting of H and halogen; wherein R₅ and R₆ may be either cis or trans;
R₈ is H;
R₉ is a moiety selected from the group consisting of H and trifluoromethylcarbonyl; or R₈ and R₉ are taken together with the core structure to form a heterocyclydiyl of formula (a): 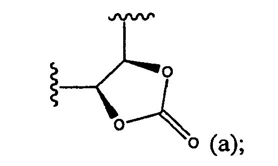
R₁₀ is a moiety selected from the group consisting of hydrogen and hydroxyl;
R₁₃ is a moiety selected from the group consisting of a C₁₋₆ alkylamino group and a C₁₋₆ alkoxy group, each of which is substituted with 0, 1, 2, or 3 hydroxyl moieties;
Y is a moiety selected from the group consisting of hydrogen, halogen and hydroxyl; or a pharmaceutically acceptable salt or ester thereof.

2. The compound of claim 1, wherein R₃ is methyl, or a pharmaceutically acceptable salt or ester thereof.

3. The compound of any of the preceding claims, wherein R₄ is selected from the group consisting of methyl and trifluoromethyl, or a pharmaceutically acceptable salt or ester thereof.

4. The compound of any of the preceding claims, wherein R₄ is methyl, or a pharmaceutically acceptable salt or ester thereof.

5. The compound of any of the preceding claims, wherein R₆ is selected from the group consisting of H and fluorine, or a pharmaceutically acceptable salt or ester thereof.

6. The compound of any of the preceding claims, wherein R₅ and R₆ are trans, or a pharmaceutically acceptable salt or ester thereof.

7. The compound of any of the preceding claims, wherein Y is selected from the group consisting of H, F, Cl and Br, or a pharmaceutically acceptable salt or ester thereof.

8. The compound of any of the preceding claims, wherein R₁₃ is an unsubstituted C₁₋₆ alkylamino, or a pharmaceutically acceptable salt or ester thereof.

9. The compound of any of the preceding claims, wherein R₁₃ is selected from the group consisting of methylamino and ethylamino, or a pharmaceutically acceptable salt or ester thereof.

10. The compound of any of the preceding claims, wherein R₁₀ is hydrogen, or a pharmaceutically acceptable salt or ester thereof.

11. The compound of any of the preceding claims, wherein said compound is selected from the group consisting of compounds having the following structures: 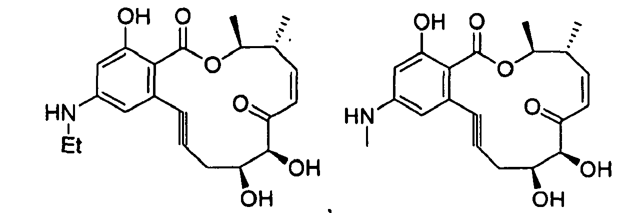 and pharmaceutically acceptable salts and esters thereof.

12. A composition comprising a compound of any of the preceding claims and a pharmaceutically acceptable carrier and optionally one or more additional therapeutic agents.

13. The composition of claim 12, wherein the composition is suitable for oral administration.

14. The composition of claim 12, wherein the composition is suitable for intravenous administration.

15. A compound of any of claims 1-11 or a composition of any of claims 12 to 14 for use in treating cancer.

16. The compound or composition for use of claim 15, wherein the cancer is a MEK1 associated cancer.

17. The compound or composition for use of claim 15 or 16, wherein the cancer is a B-RAF mutated cancer.

18. The compound or composition for use of claim 15, wherein the cancer is an NF-κB associated cancer.

19. The compound or composition for use of any of claims 15-18, wherein the compound or composition is for use in treating cancer in combination with a second chemotherapeutic drug.

20. The compound or composition for use of any of claims 15-19, wherein the compound or composition is for administration at a dosage between 0.10 mg/kg to 25 mg/kg of body weight.

21. Use of a compound of any of claims 1-11 or a composition of claim 12 in the manufacture of a medicament for the treatment of cancer.

## Patentansprüche

1. Verbindung der Formel (I) 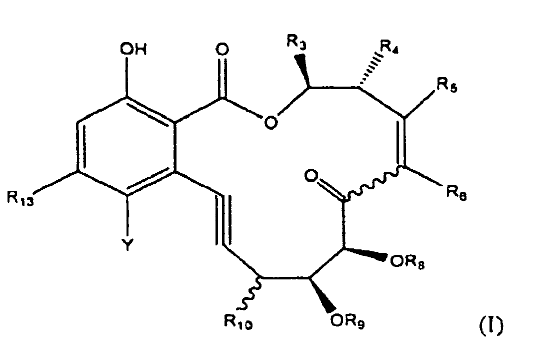 wobei
R₃ eine funktionelle Gruppe ausgewählt aus der Gruppe bestehend aus H und einer C₁₋₆-Alkylgruppe ist;
R₄ eine C₁₋₆-Alkylgruppe ist, die mit 0, 1, 2 oder 3 funktionellen Halogengruppen substituiert ist;
R₅ ein Wasserstoff ist;
R₆ eine funktionelle Gruppe ausgewählt aus der Gruppe bestehend aus H und Halogen ist;
wobei R₅ und R₆ entweder cis oder trans sein können;
R₈ H ist;
R₉ eine funktionelle Gruppe ausgewählt aus der Gruppe bestehend aus H und Trifluormethylcarbonyl ist; oder R₈ und R₉ zusammen mit der Kernstruktur genommen werden, um ein Heterocyclydil der Formel (a) zu bilden: 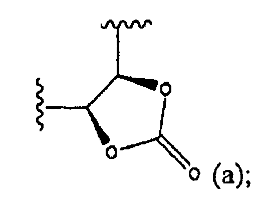
R₁₀ eine funktionelle Gruppe ausgewählt aus der Gruppe bestehend aus Wasserstoff und Hydroxyl ist;
R₁₃ eine funktionelle Gruppe ausgewählt aus der Gruppe bestehend aus einer C₁₋₆-Alkylaminogruppe und einer C₁₋₆-Alkoxygruppe ist, von denen jede mit 0, 1, 2, oder 3 funktionellen Hydroxylgruppen substituiert ist;
Y eine funktionelle Gruppe ausgewählt aus der Gruppe bestehend aus Wasserstoff, Halogen und Hydroxyl ist; oder ein pharmazeutisch akzeptables Salz oder Ester davon.

2. Verbindung von Anspruch 1, wobei R₃ Methyl oder ein pharmazeutisch akzeptables Salz oder Ester davon ist.

3. Verbindung von einem der vorhergehenden Ansprüche, wobei R₄ ausgewählt ist aus der Gruppe bestehend aus Methyl und Trifluormethyl oder einem pharmazeutisch akzeptablen Salz oder Ester davon.

4. Verbindung von einem der vorhergehenden Ansprüche, wobei R₄ Methyl oder einem pharmazeutisch akzeptablen Salz oder Ester davon ist.

5. Verbindung von einem der vorhergehenden Ansprüche, wobei R₆ ausgewählt ist aus der Gruppe bestehend aus H und Fluor oder einem pharmazeutisch akzeptablen Salz oder Ester davon.

6. Verbindung von einem der vorhergehenden Ansprüche, wobei R₅ und R₆ trans oder ein pharmazeutisch akzeptables Salz oder Ester davon sind.

7. Verbindung von einem der vorhergehenden Ansprüche, wobei Y ausgewählt ist aus der Gruppe bestehend aus H, F, Cl und Br oder einem pharmazeutisch akzeptablen Salz oder Ester davon.

8. Verbindung von einem der vorhergehenden Ansprüche, wobei R₁₃ ein nicht-substituiertes C₁₋₆-Alkylamino oder ein pharmazeutisch akzeptables Salz oder Ester davon ist.

9. Verbindung von einem der vorhergehenden Ansprüche, wobei R₁₃ ausgewählt ist aus der Gruppe bestehend aus Methylamino und Ethylamino oder einem pharmazeutisch akzeptablen Salz oder Ester davon.

10. Verbindung von einem der vorhergehenden Ansprüche, wobei R₁₀ Wasserstoff oder ein pharmazeutisch akzeptables Salz oder Ester davon ist.

11. Verbindung von einem der vorhergehenden Ansprüche, wobei die Verbindung ausgewählt ist aus der Gruppe bestehend aus Verbindungen, die folgende Strukturen aufweisen: 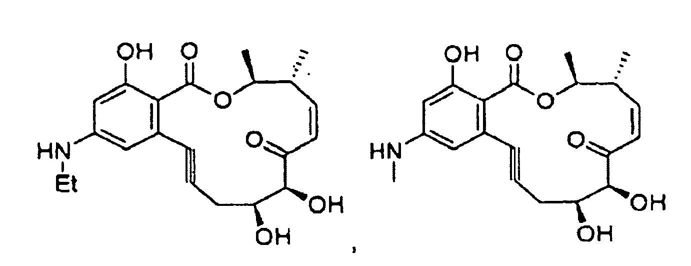 , und pharmazeutisch akzeptablen Salzen oder Estern davon.

12. Zusammensetzung, die eine Verbindung von einem der vorhergehenden Ansprüche und einen pharmazeutisch akzeptablen Träger und gegebenenfalls ein oder mehrere zusätzliche Therapeutika enthält.

13. Zusammensetzung von Anspruch 12, wobei die Zusammensetzung für die orale Verabreichung geeignet ist.

14. Zusammensetzung von Anspruch 12, wobei die Zusammensetzung für die intravenöse Verabreichung geeignet ist.

15. Verbindung von einem der Ansprüche 1-11 oder Zusammensetzung von einem der Ansprüche 12 bis 14 für die Verwendung bei der Behandlung von Krebs.

16. Verbindung oder Zusammensetzung für die Verwendung von Anspruch 15, wobei der Krebs ein MEK1-assoziierter Krebs ist.

17. Verbindung oder Zusammensetzung für die Verwendung von Anspruch 15 oder 16, wobei der Krebs ein B-RAF-mutierter Krebs ist.

18. Verbindung oder Zusammensetzung für die Verwendung von Anspruch 15, wobei der Krebs ein NF-κB-assoziierter Krebs ist.

19. Verbindung oder Zusammensetzung für die Verwendung von einem der Ansprüche 15-18, wobei die Verbindung oder Zusammensetzung für die Verwendung bei der Behandlung von Krebs in Kombination mit einem zweiten chemotherapeutischen Medikament vorgesehen ist.

20. Verbindung oder Zusammensetzung für die Verwendung von einem der Ansprüche 15-19, wobei die Verbindung oder Zusammensetzung für die Verabreichung in einer Dosierung zwischen 0,10 mg/kg bis 25 mg/kg Körpergewicht vorgesehen ist.

21. Verwendung einer Verbindung von einem der Ansprüche 1-11 oder einer Zusammensetzung von Anspruch 12 bei der Herstellung eines Medikaments für die Behandlung von Krebs.

## Revendications

1. Composé de formule (I) 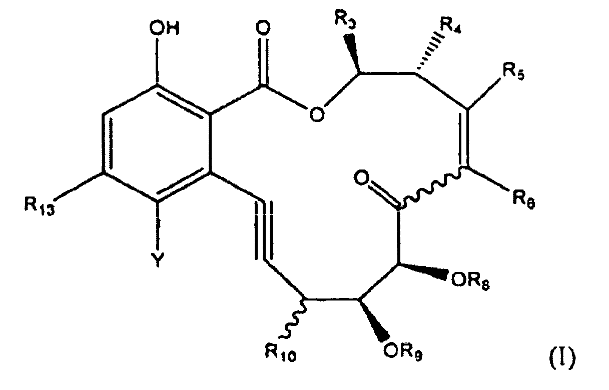 dans laquelle
R₃ est un fragment choisi dans le groupe comprenant H et un groupe alkyle en C₁ à C₆;
R₄ est un groupe alkyle en C₁ à C₆ substitué par 0, 1, 2 ou 3 fragments halogéno ;
R₅ est un atome d'hydrogène ;
R₆ est un fragment choisi dans le groupe comprenant H et un atome d'halogène ; R₅ et R₆ étant cis ou trans ;
R₈ est H ;
R₉ est un fragment choisi dans le groupe comprenant H et un groupe trifluorométhylcarbonyle ; ou R₈ et R₉ sont pris conjointement avec la structure centrale pour former un groupe hétérocyclydiyle de formule (a): 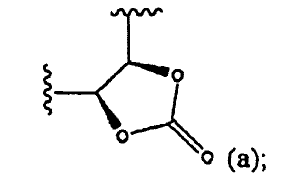
R₁₀ est un fragment choisi dans le groupe comprenant un atome d'hydrogène et un groupe hydroxyle ;
R₁₃ est un fragment choisi dans le groupe comprenant un groupe alkylamino en C₁ à C₆ ou un groupe alcoxy en C₁ à C₆, chacun d'eux étant substitué par 0, 1, 2 ou 3 fragments hydroxyle ;
Y est un fragment choisi dans le groupe comprenant un atome d'hydrogène, d'halogène et un groupe hydroxyle ; ou un sel ou ester pharmaceutiquement acceptable de ceux-ci.

2. Composé selon la revendication 1, dans lequel R₃ est un groupe méthyle ou un sel ou ester pharmaceutiquement acceptable de celui-ci.

3. Composé selon l'une quelconque des revendications précédentes, dans lequel R₄ est choisi dans le groupe comprenant un groupe méthyle et trifluorométhyle ou un sel ou ester pharmaceutiquement acceptable de ceux-ci.

4. Composé selon l'une quelconque des revendications précédentes, dans lequel R₄ est un groupe méthyle ou un sel ou ester pharmaceutiquement acceptable de celui-ci.

5. Composé selon l'une quelconque des revendications précédentes, dans lequel R₆ est choisi dans le groupe comprenant H et un atome de fluor ou un sel ou ester pharmaceutiquement acceptable de ceux-ci.

6. Composé selon l'une quelconque des revendications précédentes, dans lequel R₅ et R₆ sont trans ou un sel ou ester pharmaceutiquement acceptable de ceux-ci.

7. Composé selon l'une quelconque des revendications précédentes, dans lequel Y est choisi dans le groupe comprenant H, F, Cl et Br, ou un sel ou ester pharmaceutiquement acceptable de ceux-ci.

8. Composé selon l'une quelconque des revendications précédentes, dans lequel R₁₃ est un groupe alkylamino en C₁ à C₆ non substitué ou un sel ou ester pharmaceutiquement acceptable de celui-ci.

9. Composé selon l'une quelconque des revendications précédentes, dans lequel R₁₃ est choisi dans le groupe comprenant les groupes méthylamino et éthylamino ou un sel ou ester pharmaceutiquement acceptable de ceux-ci.

10. Composé selon l'une quelconque des revendications précédentes, dans lequel R₁₀ est un atome d'hydrogène un sel ou ester pharmaceutiquement acceptable de celui-ci.

11. Composé selon l'une quelconque des revendications précédentes, dans lequel ledit composé est choisi dans le groupe comprenant des composés ayant les structures suivantes : 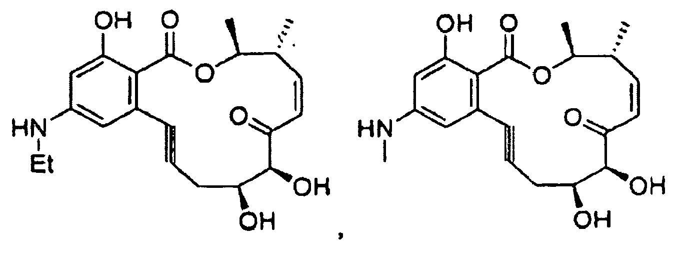 un sel ou ester pharmaceutiquement acceptable de ceux-ci.

12. Composition comprenant un composé selon l'une quelconque des revendications précédentes et un véhicule pharmaceutiquement acceptable et éventuellement un ou plusieurs autres agents thérapeutiques.

13. Composition selon la revendication 12, ladite composition étant appropriée pour l'administration orale.

14. Composition selon la revendication 12, ladite composition étant appropriée pour l'administration intraveineuse.

15. Composé selon l'une quelconque des revendications 1 à 11, ou composition selon l'une quelconque des revendications 12 à 14, destiné à être utilisé dans le traitement du cancer.

16. Composé ou composition à utiliser selon la revendication 15, le cancer étant un cancer associé à MEK1.

17. Composé ou composition à utiliser selon la revendication 15 ou 16, le cancer étant un cancer B-RAF muté.

18. Composé ou composition à utiliser selon la revendication 15, le cancer étant un cancer associé à NF-κB.

19. Composé ou composition à utiliser selon l'une quelconque des revendications 15 à 18, le composé ou la composition étant destiné(e) à être utilisé(e) dans le traitement du cancer en combinaison avec un deuxième médicament chimiothérapeutique.

20. Composé ou composition à utiliser selon l'une quelconque des revendications 15 à 19, le composé ou la composition étant destiné(e) à l'administration en une dose entre 0,10 mg/kg à 25 mg/kg de poids corporel.

21. Utilisation d'un composé selon l'une quelconque des revendications 1 à 11, ou d'une composition selon la revendication 12, dans la production d'un médicament pour le traitement du cancer.
